# EUROPEAN PATENT SPECIFICATION

(11) **EP 3 781 713 B1**
(45) Date of publication and mention of the grant of the patent: **18.03.2026**
(21) Application number: 19788266.5
(22) Date of filing: 15.04.2019
(51) Int. Cl.: G16B 20/00, G16B 20/10, C12Q 1/6806, C12Q 1/6886, G16B 20/20, G16B 40/20

(54) **SYSTEMS AND METHODS FOR DETECTING CANCER VIA CFDNA SCREENING**
SYSTEME UND VERFAHREN ZUR KREBSERKENNUNG MITTELS CFDNA-SCREENING
SYSTÈMES ET PROCÉDÉS DE DÉTECTION D'UN CANCER PAR L'INTERMÉDIAIRE D'UN CRIBLAGE D'ADNCF

(30) Priority: 16.04.2018 US 201862658489 P
(43) Date of publication of application: 24.02.2021
(73) Proprietor: Memorial Sloan-Kettering Cancer Center, New York, NY 10065 (US)
(72) Inventor: BERGER, Michael, New York 10065 (US); TSUI, Dana, New York 10065 (US); SRINIVASAN, Preethi, New York 10065 (US); PATEL, Juber, New York 10065 (US); HOUCK-LOOMIS, Brian, New York 10065 (US); HASAN, Maysun, New York 10065 (US); MENG, Fanli, New York 10065 (US); STADLER, Zsofia, New York 10065 (US)
(74) Representative: Barker Brettell LLP
(86) International application number: PCT/US2019/027487
(87) International publication number: WO 2019/204208

(56) References cited:
- WO-A1-2018/064629
- US-A1- 2015 368 708
- US-A1- 2016 319 345
- US-A1- 2017 213 008
- US-A1- 2018 089 373
- XIA LIGANG ET AL: "Statistical analysis of mutant allele frequency level of circulating cell-free DNA and blood cells in healthy individuals", vol. 7, no. 1, 1 December 2017 (2017-12-01), XP055868439, Retrieved from the Internet <URL:https://www.nature.com/articles/s41598-017-06106-1.pdf> DOI: 10.1038/s41598-017-06106-1
- MUKHERJEE INDRANEIL ET AL: "Available technologies and clinical applications of targeted chemotherapy in pancreatic cancer", CANCER GENETICS, ELSEVIER, AMSTERDAM, NL, vol. 209, no. 12, 5 August 2016 (2016-08-05), pages 582 - 591, XP029827920, ISSN: 2210-7762, DOI: 10.1016/J.CANCERGEN.2016.08.001
- RAZAVI PEDRAM ET AL: "High-intensity sequencing reveals the sources of plasma circulating cell-free DNA variants", NATURE MEDICINE, NATURE PUBLISHING GROUP US, NEW YORK, vol. 25, no. 12, 25 November 2019 (2019-11-25), pages 1928 - 1937, XP036953312, ISSN: 1078-8956, [retrieved on 20191125], DOI: 10.1038/S41591-019-0652-7

## Description

### CROSS-REFERENCE TO RELATED APPLICATIONS

This application claims the benefit of and priority to U.S. provisional Patent Application No. 62/658,489, filed on April 16, 2018.

### FIELD OF THE DISCLOSURE

The present disclosure is generally directed to processing data to identify cancer-related mutations and microsatellite instability in cell-free DNA (cfDNA) sequence data.

### BACKGROUND OF THE DISCLOSURE

The following description of the background of the present technology is provided simply as an aid in understanding the present technology and is not admitted to describe or constitute prior art to the present technology.

Tumors continually shed DNA into the circulation (circulating tumor DNA, or ctDNA), where it is readily accessible (Stroun et al., Eur J Cancer Clin Oncol 23:707-712 (1987)). Analysis of such cancer-derived cell-free DNA (cfDNA) has the potential to revolutionize cancer detection, tumor genotyping, and disease monitoring. For example, noninvasive access to tumor-derived DNA *via* liquid biopsies is particularly attractive for solid tumors. However, in most early- and many advanced-stage solid tumors, ctDNA blood levels are extremely low (~0.1%) (Bettegowda, C. et al., Sci. Transl. Med. 6:224ra24 (2014); Newman, A.M. et al., Nat. Med. 20:548-554 (2014)), thus complicating ctDNA detection and analysis. Mutation fractions in cfDNA are often lower than those observed in tissue samples from the same subject and may approach the noise levels of next-generation sequencing workflows, making it impossible to distinguish true somatic mutations from artifacts. Recovery of cfDNA molecules and non-biological errors introduced during library preparation and sequencing limit analytical sensitivity and continue to represent a major obstacle for ultrasensitive ctDNA profiling.

Statistical analysis of mutant allele frequency level of circulating cell-free DNA and blood cells in healthy individuals has been disclosed in Xia, L., Li, Z., Zhou, B. et al. Statistical analysis of mutant allele frequency level of circulating cell-free DNA and blood cells in healthy individuals. Sci Rep 7, 7526 (2017). https://doi.org/10.1038/s41598-017-06106-1.

US 2018/089373 A1 discloses a method for qualifying a subject for a subset of therapies using medical history data and biologic data.

### SUMMARY

The invention is as defined in the appended claim set.

The present disclosure is directed to more sensitive and high-throughput systems and methods for effective detection of somatic mutations and microsatellite instability from cfDNA, particularly for early-stage cancer subjects.

In one aspect, the disclosure is related to a computer-implemented method. The method includes receiving, by one or more processors, from a next generation sequencing device (i) a plurality of nucleic acid (*e.g.,* cell-free DNA (cfDNA)) sequence read-pairs derived from a subject, each nucleic acid (*e.g.,* cfDNA) sequence read from the plurality of nucleic acid (*e.g.,* cfDNA) sequence reads including either a forward unique molecular identifier (UMI) or a reverse UMI, and (ii) a plurality of white blood cell (WBC)-derived sequence read-pairs derived from the subject, each WBC-derived sequence read from the plurality of WBC-derived sequence reads optionally including the forward UMI or the reverse UMI. The method further includes for each microsatellite locus of a plurality of microsatellite loci. The method also includes identifying, by the one or more processors, a first subset of the plurality of nucleic acid (*e.g.,* cfDNA) sequence reads and a second subset of the plurality of WBC-derived sequence reads, each read in the first subset and the second subset corresponds to the microsatellite locus. The method further includes identifying, by the one or more processors, from the first subset and the second subset, a set of alleles, each allele of the set of alleles having a distinct sequence. The method also includes determining, by the one or more processors, for each allele of the set of alleles, a number of nucleic acid *(e.g.,* cfDNA) sequence reads that include the allele. The method further includes determining, by the one or more processors, for each allele of the set of alleles, a number of WBC-derived sequence reads that include the allele. The method also includes determining, by the one or more processors, for each allele in the set of alleles, an absolute difference based on a difference between the number of nucleic acid (*e.g.,* cfDNA) sequence reads for the allele and the number of WBC-derived sequence reads for the allele. The method also includes determining, by the one or more processors, for each microsatellite locus from the plurality of microsatellite loci, a distance based on a sum of absolute differences associated with all alleles in the set of alleles. The method further includes generating, by the one or more processors, a first distribution indicating a number of microsatellite loci having distances within a group of distinct distance intervals. The method further includes generating, by the one or more processors, a second distribution indicating a number of microsatellite loci having distances within the group of distinct distance intervals, the second distribution derived from distances associated with each microsatellite locus of the plurality of microsatellite loci observed in a reference sample. The method also includes determining, by the one or more processors, that a number of microsatellite loci in the first distribution above a threshold distance metric is greater than a number of microsatellite loci in the second distribution above the threshold distance metric to detect a presence of microsatellite instability in the subject. The method additionally includes storing, by the one or more processors, responsive to the determination, in one or more data structures, an association between the subject and the presence of microsatellite instability.

In some embodiments, the method further includes normalizing, by the one or more processors, for each allele of the set of alleles, the number of nucleic acid (*e.g.,* cfDNA) sequence reads that include the allele based on a sum of the number of nucleic acid (*e.g.,* cfDNA) sequence reads corresponding to all alleles in the set of alleles to generate a respective normalized number of nucleic acid (*e.g.,* cfDNA) sequence reads corresponding to the allele, and normalizing, by the one or more processors, for each allele of the set of alleles, the number of WBC-derived sequence that include the allele based on a sum of the number of WBC-derived sequence reads corresponding to all alleles in the set of alleles to generate a respective normalized number of WBC-derived sequence reads corresponding to the allele, where, for each allele in the set of alleles, the absolute difference is based on a difference between the normalized number of nucleic acid (*e.g.,* cfDNA) sequence reads for the allele and the normalized number of WBC-derived sequence reads for the allele.

In some embodiments, wherein the sum of absolute differences associated with all alleles in the set of alleles is based on a sum of an absolute difference between normalized number of cfDNA sequence reads and normalized number of WBC-derived sequence reads for each allele in the set of alleles. In some embodiments, wherein the subject suffers from, or is suspected of having Lynch Syndrome. In some embodiments, the subject harbors at least one mutation in one or more mismatch repair genes selected from the group consisting of MSH2, MSH6, MLH1, and PMS2. In some embodiments, the subject suffers from or is at risk for ovarian cancer, breast cancer, colorectal cancer, lung cancer, prostate cancer, gastric cancer, pancreatic cancer, cervical cancer, liver cancer, bladder cancer, cancer of the urinary tract, thyroid cancer, renal cancer, carcinoma, melanoma, head and neck cancer, or brain cancer. In some embodiments, the method further includes determining the presence of at least one mutation in an exon of a cancer-related gene selected from the group consisting of: AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3, GNA11, GNAQ, GNAS, H3F3A, HIST1H3B, HRAS, IDH1, IDH2, IKZF1, INPPL1, JAK1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL, and XPO1.

In some embodiments, the at least one mutation is a deletion, an insertion, a translocation, an inversion, a copy number variant, or a point mutation. In some embodiments, the method further includes determining the presence of at least one genomic alteration in an intron of a cancer-related gene selected from the group consisting of: ALK, BRAF, EGFR. ETV6, FGFR2, FGFR3, MET, NTRK1, RET and ROS1 or a promoter region of TERT. In some embodiments, the subject lacks detectable tumors.

In another aspect, the disclosure is related to a method for determining the efficacy of a therapy in a subject with a MSI-High tumor. The method includes administering the therapy to the subject. The method further includes detecting the presence of microsatellite instability in a first nucleic acid (*e.g.,* cfDNA) sample obtained from the subject using any of the computer-implemented methods disclosed herein, following administration of the therapy. The method also includes determining that the therapy is effective when the first nucleic acid (*e.g.,* cfDNA) sample shows a shift towards a distance metric that is associated with microsatellite stability (MSS) compared to that observed in a control sample obtained from the subject prior to administration of the therapy.

In some embodiments, the therapy is one or more of radiation therapy, chemotherapy, surgery, immunotherapy, or surgery. In some embodiments, chemotherapy includes the administration of one or more chemotherapeutic agents selected from the group consisting of abraxane, capecitabine, erlotinib, fluorouracil (5-FU), gemcitabine, irinotecan, leucovorin, nab-paclitaxel, cisplatin, irinotecan, docetaxel, oxaliplatin, tipifarnib, everolimus, sunitinib, dovitinib, ruxolitinib, pegylated-hyaluronidase, pemetrexed, folinic acid, paclitaxel, MK2206, GDC-0449, IPI-926, gamma secretase/RO4929097, M402, and LY293111. In some embodiments, immunotherapy includes the administration of one or more agents selected from the group consisting of immune checkpoint inhibitors (e.g., antibodies targeting CTLA-4, PD-1, PD-L1), ipilimumab, 90Y-Clivatuzumab tetraxetan, pembrolizumab, nivolumab, trastuzumab, cixutumumab, ganitumab, demcizumab, cetuximab, nimotuzumab, dalotuzumab, sipuleucel-T, CRS-207, and GVAX.

In another aspect, the disclosure is related to a system including one or more processors. The one or more processors are configured to receive from a next generation sequencing device (i) a plurality of nucleic acid (*e.g.,* cfDNA) sequence read-pairs derived from a subject, each nucleic acid (*e.g.,* cfDNA) sequence read from the plurality of nucleic acid (*e.g.,* cfDNA) sequence reads including either a forward unique molecular identifier (UMI) or a reverse UMI, and (ii) a plurality of WBC-derived sequence read-pairs derived from the subject, each WBC-derived sequence read from the plurality of WBC-derived sequence reads optionally including the forward UMI or the reverse UMI. The one or more processors are configured to, for each microsatellite locus of a plurality of microsatellite loci, identify a first subset of the plurality of nucleic acid (*e.g.,* cfDNA) sequence reads and a second subset of the plurality of WBC-derived sequence reads, each read in the first subset and the second subset corresponds to the microsatellite locus, identify from the first subset and the second subset, a set of alleles, each allele of the set of alleles having a distinct sequence, determine, for each allele of the set of alleles, a number of nucleic acid (*e.g.,* cfDNA) sequence reads that include the allele, determine, for each allele of the set of alleles, a number of WBC-derived sequence reads that include the allele, determine, for each allele in the set of alleles, an absolute difference based on a difference between the number of nucleic acid (*e.g.,* cfDNA) sequence reads for the allele and the number of WBC-derived sequence reads for the allele. The one or more processors are configured to determine, for each microsatellite locus from the plurality of microsatellite loci, a distance based on a sum of absolute differences associated with all alleles in the set of alleles. The one or more processors are configured to generate a first distribution indicating a number of microsatellite loci having distances within a group of distinct distance intervals. The one or more processors are configured to generate a second distribution indicating a number of microsatellite loci having distances within the group of distinct distance intervals, the second distribution derived from distances associated with each microsatellite locus of the plurality of microsatellite loci observed in a reference sample. The one or more processors are configured to determine that a number of microsatellite loci in the first distribution above a threshold distance metric is greater than a number of microsatellite loci in the second distribution above the threshold distance metric to detect a presence of microsatellite instability in the subject. The one or more processors are configured to store, responsive to the determination, in one or more data structures, an association between the subject and the presence of microsatellite instability.

In some embodiments, the one or more processors are configured to normalize, for each allele of the set of alleles, the number of nucleic acid (*e.g.,* cfDNA) sequence reads that include the allele based on a sum of the number of nucleic acid (*e.g.,* cfDNA) sequence reads corresponding to all alleles in the set of alleles to generate a respective normalized number of nucleic acid (*e.g.,* cfDNA) sequence reads corresponding to the allele, and normalize, for each allele of the set of alleles, the number of WBC-derived sequence that include the allele based on a sum of the number of WBC-derived sequence reads corresponding to all alleles in the set of alleles to generate a respective normalized number of WBC-derived sequence reads corresponding to the allele, where, for each allele in the set of alleles, the absolute difference is based on a difference between the normalized number of nucleic acid (*e.g.,* cfDNA) sequence reads for the allele and the normalized number of WBC-derived sequence reads for the allele.

In one or more embodiments, the one or more processors are configured to generate a machine-learning or statistical classifier that generates a decision boundary on a coordinate space that separates a first set of data points that represent presence of microsatellite instability in sequence reads and a second set of data points that represent no presence of microsatellite instability in sequence reads, process the first distribution using the classifier to determine whether the first distribution belongs to the first set of data points or to the second set of data points, determine microsatellite instability responsive to the classifier classifying the first distribution as belonging to the first set of data points that represent presence of microsatellite instability.

In another aspect, the disclosure is related to a computer-implemented method to identify at least one mutation in cell free DNA (cfDNA) present in a sample processed by a next-generation sequencing device. The method includes receiving, by a computer server including one or more processors, from the next generation sequencing device a plurality of first cfDNA sequence reads derived from one strand of a template double-stranded cfDNA molecule (hereby referred to as 'sense' strand), each cfDNA sequence read from the plurality of first cfDNA sequence reads including a first unique molecular identifier (UMI), and a plurality of second cfDNA sequence reads derived from the opposite (complementary) strand of the template double-stranded cfDNA molecule (hereby referred to as 'antisense' strand), each cfDNA sequence read from the plurality of second cfDNA sequence reads including a second UMI. The method further includes, identifying, by the computer server, a first set of mutations in each of the plurality of first cfDNA sequence reads. The method also includes identifying, by the computer server, a second set of mutations in each of the plurality of second cfDNA sequence reads. The method also includes identifying a first set of consensus mutations in the plurality of first cfDNA sequence reads, the first set of consensus mutations including mutations from the first set of mutations that appear in the same position in the respective cfDNA sequence read of the plurality of first cfDNA sequence reads. The method further includes identifying a second set of consensus mutations in the plurality of second cfDNA sequence reads, the second set of consensus mutations including mutations from the second set of mutations that appear in the same position in the respective cfDNA sequence reads of the plurality of second cfDNA sequence reads. The method further includes identifying a third set of consensus mutations selected from the first set of consensus mutations, each mutation in the third set of consensus mutations having a consistent mutation in the second set of consensus mutations. The method also includes identifying a WBC set of mutations in a plurality of white blood cell (WBC) sequence reads derived from the subject. The method additionally includes generating a final set of consensus mutations by removing from the third set of consensus mutations those consensus mutations that appear in the set of WBC mutations.

In some embodiments, the cfDNA in the sample comprises circulating tumor DNA (ctDNA). In some embodiments, the at least one mutation identified is in an exon of a cancer-related gene selected from the group consisting of:
AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3, GNA11, GNAQ, GNAS, H3F3A, HIST1H3B, HRAS, IDH1, IDH2, IKZF1, INPPL1, JAK1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL, and XPO1.
In some embodiments, the at least one genomic alteration detected is in an intron of a cancer-related gene selected from the group consisting of: ALK, BRAF, EGFR. ETV6, FGFR2, FGFR3, MET, NTRK1, RET and ROS1 or a promoter region of TERT. In some embodiments, the at least one mutation detected is in a microsatellite locus for microsatellite instability. In some embodiments, at least one mutation detected is in cancer-related gene selected from the group consisting of: BRCA1/2, MLH1, MSH2, MSH6, PMS2. In some embodiments, the at least one mutation is a deletion, an insertion, a translocation, an inversion, a copy number variant, or a point mutation. In some embodiments, the cfDNA sample is serum, plasma, sweat, tears, urine, saliva, synovial fluid, lymphatic fluid, ascites fluid, amniotic fluid, or interstitial fluid. In some embodiments, the subject suffers from or is at risk for ovarian cancer, breast cancer, colorectal cancer, lung cancer, prostate cancer, gastric cancer, pancreatic cancer, cervical cancer, liver cancer, bladder cancer, cancer of the urinary tract, thyroid cancer, renal cancer, carcinoma, melanoma, head and neck cancer, or brain cancer.

In some embodiments, the method further includes trimming the forward cfDNA UMI from the plurality of first cfDNA sequence reads and trimming the second cfDNA UMI from the plurality of second cfDNA sequence reads prior to identifying the first set of mutations and the second set of mutations. In some embodiments, the method further includes filtering the first set of mutations and the second set of mutations based on known hotspot mutations. In some embodiments, the method also includes filtering the first set of mutations and the second set of mutations based on a set of mutations identified in cfDNA sequence reads associated with healthy individuals. In some embodiments, the method also includes identifying the first set of consensus mutations in the plurality of first cfDNA sequence reads, the first set of consensus mutations including mutations from the first set of mutations that appear in the same position in more than half of the respective cfDNA sequence reads of the plurality of first cfDNA sequence reads. In some embodiments, the method further includes identifying the second set of consensus mutations in the plurality of second cfDNA sequence reads, the second set of consensus mutations including mutations from the second set of mutations that appear in the same position in more than half of the respective cfDNA sequence reads of the plurality of second cfDNA sequence reads.

In some embodiments, the method further includes receiving, by the computer server including one or more processors, from the next generation sequencing device a plurality of first WBC sequence reads derived from the subject, each WBC sequence read from the plurality of first WBC sequence reads optionally including a first WBC UMI and a plurality of second WBC sequence reads derived from the subject, each WBC sequence read from the plurality of second cfDNA sequence reads optionally including a second WBC UMI. The method also includes identifying, by the computer server, a first WBC set of mutations in each of the plurality of first WBC sequence reads. The method further includes identifying, by the computer server, a second WBC set of mutations in each of the plurality of second WBC sequence reads. The method also includes identifying a first WBC set of consensus mutations in the plurality of first WBC sequence reads, the first set of consensus WBC mutations including mutations from the first WBC set of mutations that appear in the same position in the respective WBC sequence reads of the plurality of first WBC sequence reads. The method also includes identifying a second WBC set of consensus mutations in the plurality of second WBC sequence reads, the second set of consensus WBC mutations including mutations from the second WBC set of mutations that appear in the same position in the respective WBC sequence reads of the plurality of second WBC sequence reads. The method further includes identifying the WBC set of mutations selected from the first WBC set of consensus mutations, each mutation in the WBC set of mutations having a consistent mutation in the second WBC set of consensus mutations. In some embodiments, having the consistent mutation in the second set of consensus mutations includes a nucleotide sequence that is complementary to a nucleotide sequence of the corresponding consensus mutation in the first set of consensus mutation.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1A is a block diagram depicting an embodiment of a network environment comprising a client device in communication with server device.
FIG. 1B is a block diagram depicting a cloud computing environment comprising client device in communication with cloud service providers.
FIGS. 1C and 1D are block diagrams depicting embodiments of computing devices useful in connection with the methods and systems described herein.
FIG. 2 illustrates cfDNA strands with attached duplex UMIs and sample barcodes.
FIG. 3 illustrates a flow diagram of a mutation identification process 300.
FIG. 4 illustrates exemplary sense strand cfDNA and anti-sense strand cfDNA sequence read-pairs including UMIs and sample barcodes to determine consensus mutations.
FIG. 5A illustrates the frequency of sample barcode mis-assignment that occurs with or without the use of duplex UMIs.
FIG. 5B illustrates how dual index sequencing with UMIs decreases the frequency of sample barcode mis-assignment in sequence reads.
FIG. 6A shows the % noise level observed when cfDNA sequence data derived from subject samples are either not processed or processed using the Picard software (Broad Institute, Cambridge MA). The initial subject samples comprised either 10 ng or 30 ng cfDNA and were subjected to next-generation sequencing.
FIG. 6B shows an example of the % noise level observed when cfDNA sequence data derived from subject samples are processed using the data processing methods of the present disclosure.
FIG. 7A illustrates an example of the family size distribution of the cfDNA sequence reads observed when using the data processing methods of the present disclosure. The cfDNA sequence reads are derived from subject samples comprising either 10 ng or 30 ng cfDNA.
FIG. 7B illustrates an example of the collapsed coverage of cfDNA sequence reads observed when using the data processing methods of the present disclosure. The cfDNA sequence reads are derived from subject samples comprising either 10 ng or 30 ng cfDNA.
FIG. 7C shows an example of the fractions of various family types of cfDNA sequence reads observed when using the data processing methods of the present disclosure. The cfDNA sequence reads are derived from subject samples comprising either 10 ng or 30 ng cfDNA.
FIG. 8A shows the correlation between the minor allele frequency (MAF) observed using the data processing methods disclosed herein and the MAF observed using a different (orthogonal) screening method.
FIG. 8B illustrates an example of the variant calling results achieved with the cfDNA data processing methods disclosed herein compared to the MSK IMPACT NGS method on tissue and whole blood samples from the same patient (Cheng et al., J. Mol. Diagnostics 17(3): 251-264 (2015)).
FIG. 8C illustrates that the cfDNA data processing methods disclosed herein correctly identified that PIK3CA E542K and E545K mutations occur in two separate DNA molecules. The presence of the mutations was confirmed using droplet digital PCR.
FIG. 9 shows the landscape of microsatellite instability (MSI) observed in different cancers. MSI data was obtained from a large number of advanced cancer subjects that were screened by the MSK IMPACT method (Middha et al., JCO Precision Oncology (2017)).
FIG. 10 shows the MSIsensor results of seven plasma cfDNA samples sequenced using MSK-IMPACT that were obtained from MSI-High subjects (as previously determined by MSK-IMPACT assay for tumor tissue). Only one sample showed a high degree of tumor-derived cfDNA in plasma sufficient to call MSI.
FIG. 11 shows that MSIsensor in its current form failed to adequately discriminate between MSI-High and MSS (microsatellite stable) cases when analyzing cfDNA data.
FIG. 12 shows an exemplary comparison of the number of individual sequence reads observed for every possible allele (1 to N) at a microsatellite locus between a tumor sample and a matched normal control sample (adapted from Gonzales, R et al. Current applications of molecular pathology in colorectal carcinoma. Applied Cancer Research 37:13 (2017)).
FIG. 13 shows a flow diagram of an example process for determining the presence of microsatellite instability in cfDNA samples.
FIG. 14A shows an exemplary distribution of computed allelic distances for a single MSI tumor sample and a single MSS tumor sample. FIG. 14B shows an exemplary distribution of computed allelic distances averaged across 26,000 tumor samples.
FIG. 15 shows an exemplary distribution of computed allelic distances for 7 plasma cfDNA samples from subjects with MSS tumors (gray) and 12 plasma cfDNA samples from subjects with MSI tumors (black).
FIG. 16 shows an example of a decision boundary generated by a SVM classifier that is useful for accurately discriminating between MSI and MSS cfDNA samples.
FIG. 17A-17B show a summary of the ctDNA results of a subject treated with pembrolizumab/radiation at three distinct time points. The subject was a 32-year-old male diagnosed with Stage III-C rectal cancer and Lynch Syndrome (MSH6 p.Tyr524Glnfs*6). The subject was previously treated with FOLFOX (i.e., folinic acid (a.k.a., leucovorin, FA or calcium folinate), fluorouracil (5FU), and oxaliplatin) and had a tumor MSISensor Score of 42.04 prior to treatment with pembrolizumab/radiation.
FIG. 18A-18B show a summary of the ctDNA results of a subject treated with pembrolizumab at three distinct time points. The subject was a 23-year-old male diagnosed with Stage III-C rectal cancer and Lynch Syndrome (MLH1 c.1990-1G>C). The subject was previously treated with capecitabin and radiation and had a tumor MSISensor Score of 34.37 prior to treatment with pembrolizumab.

### DETAILED DESCRIPTION

For purposes of reading the description of the various embodiments below, the following descriptions of the sections of the specification and their respective contents may be helpful:
Section A describes a network environment and computing environment which may be useful for practicing embodiments described herein.
Section B describes embodiments of systems and methods for identifying mutations in cell-free DNA.
Section C describes embodiments of systems and methods for detecting the presence of microsatellite instability in cell-free DNA.

The superior performance of the methods and systems disclosed herein with respect to detecting microsatellite instability in cfDNA may be attributed, at least in part to, the following technical features:
(a) Normalization of allelic coverage at the sample level as well as the microsatellite level, which helps mitigate inaccuracies caused by differences in coverage across samples and genomic regions;
(b) Absolute distance associated with each microsatellite locus is a more robust estimate that is resistant to outliers and suitable for sparse data;
(c) Support Vector Machine (SVM) classifiers increase computational efficiency and are naturally resistant to overfitting; and
(d) Leveraging upstream collapsing and error suppression allows for highly accurate quantification of MSI.

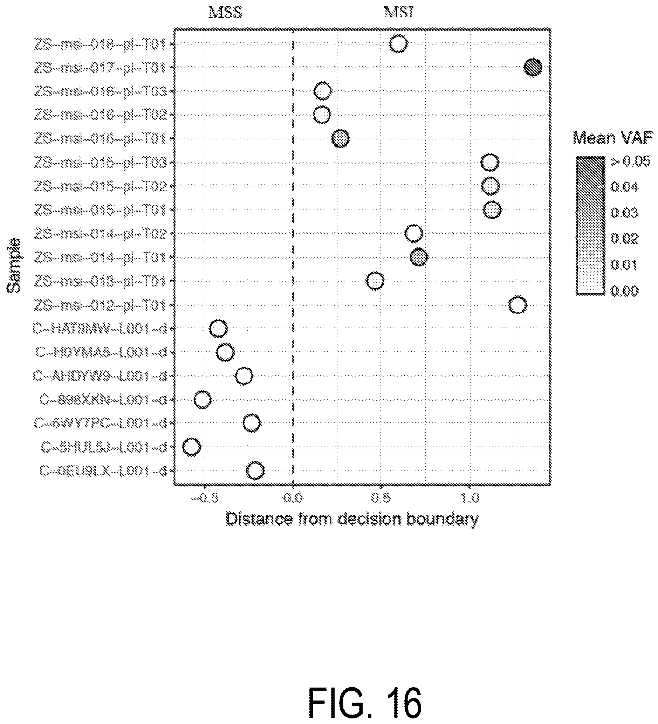

The methods disclosed herein permit early detection of cancer in high-risk subjects, such as Lynch Syndrome, and can be used as an indicator of responsiveness to a particular therapeutic regimen. MSI detection is a critical component of clinical genomic profiling to guide diagnosis and treatment selection. Moreover, as shown in FIGs. 16-18, MSI detection appears to be more sensitive than mutations in cancer-related genes. For instance, MSI is apparent in tumors with no detectable mutations, thus making it a more sensitive biomarker of occult metastatic disease (i.e., minimal residual disease).

### A. Computing and Network Environment

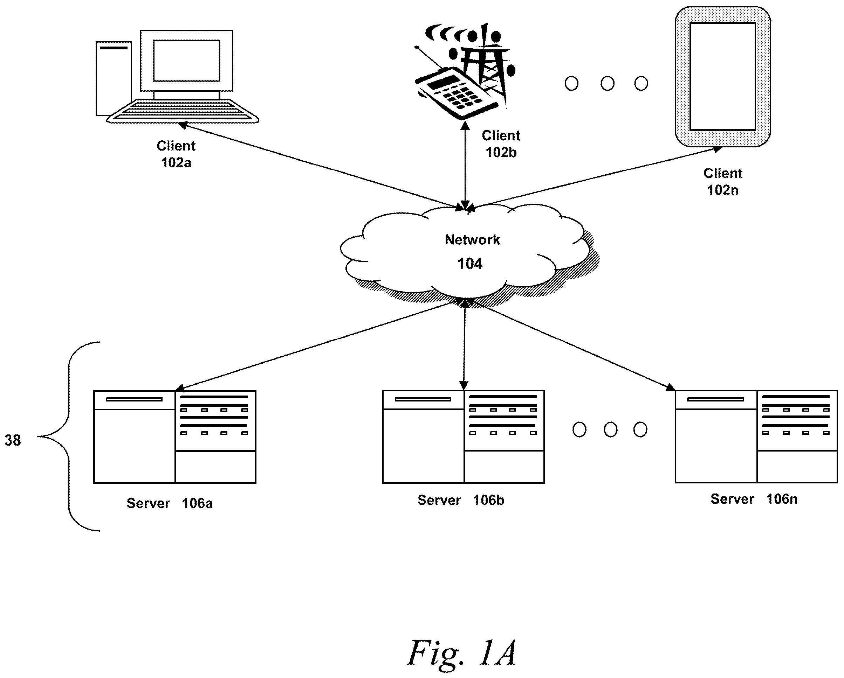

Prior to discussing specific embodiments of the present solution, it may be helpful to describe aspects of the operating environment as well as associated system components (e.g., hardware elements) in connection with the methods and systems described herein. Referring to FIG. 1A, an embodiment of a network environment is depicted. In brief overview, the network environment includes one or more clients 102a-102n (also generally referred to as local machine(s) 102, client(s) 102, client node(s) 102, client machine(s) 102, client computer(s) 102, client device(s) 102, endpoint(s) 102, or endpoint node(s) 102) in communication with one or more servers 106a-106n (also generally referred to as server(s) 106, node 106, or remote machine(s) 106) via one or more networks 104. In some embodiments, a client 102 has the capacity to function as both a client node seeking access to resources provided by a server and as a server providing access to hosted resources for other clients 102a-102n.

Although FIG. 1A shows a network 104 between the clients 102 and the servers 106, the clients 102 and the servers 106 may be on the same network 104. In some embodiments, there are multiple networks 104 between the clients 102 and the servers 106. In one of these embodiments, a network 104' (not shown) may be a private network and a network 104 may be a public network. In another of these embodiments, a network 104 may be a private network and a network 104' a public network. In still another of these embodiments, networks 104 and 104' may both be private networks.

The network 104 may be connected via wired or wireless links. Wired links may include Digital Subscriber Line (DSL), coaxial cable lines, or optical fiber lines. The wireless links may include BLUETOOTH, Wi-Fi, Worldwide Interoperability for Microwave Access (WiMAX), an infrared channel or satellite band. The wireless links may also include any cellular network standards used to communicate among mobile devices, including standards that qualify as 1G, 2G, 3G, or 4G. The network standards may qualify as one or more generation of mobile telecommunication standards by fulfilling a specification or standards such as the specifications maintained by International Telecommunication Union. The 3G standards, for example, may correspond to the International Mobile Telecommunications-2000 (IMT-2000) specification, and the 4G standards may correspond to the International Mobile Telecommunications Advanced (IMT-Advanced) specification. Examples of cellular network standards include AMPS, GSM, GPRS, UMTS, LTE, LTE Advanced, Mobile WiMAX, and WiMAX-Advanced. Cellular network standards may use various channel access methods e.g. FDMA, TDMA, CDMA, or SDMA. In some embodiments, different types of data may be transmitted via different links and standards. In other embodiments, the same types of data may be transmitted via different links and standards.

The network 104 may be any type and/or form of network. The geographical scope of the network 104 may vary widely and the network 104 can be a body area network (BAN), a personal area network (PAN), a local-area network (LAN), e.g. Intranet, a metropolitan area network (MAN), a wide area network (WAN), or the Internet. The topology of the network 104 may be of any form and may include, e.g., any of the following: point-to-point, bus, star, ring, mesh, or tree. The network 104 may be an overlay network which is virtual and sits on top of one or more layers of other networks 104'. The network 104 may be of any such network topology as known to those ordinarily skilled in the art capable of supporting the operations described herein. The network 104 may utilize different techniques and layers or stacks of protocols, including, e.g., the Ethernet protocol, the internet protocol suite (TCP/IP), the ATM (Asynchronous Transfer Mode) technique, the SONET (Synchronous Optical Networking) protocol, or the SDH (Synchronous Digital Hierarchy) protocol. The TCP/IP internet protocol suite may include application layer, transport layer, internet layer (including, e.g., IPv6), or the link layer. The network 104 may be a type of a broadcast network, a telecommunications network, a data communication network, or a computer network.

In some embodiments, the system may include multiple, logically-grouped servers 106. In one of these embodiments, the logical group of servers may be referred to as a server farm 38 or a machine farm 38. In another of these embodiments, the servers 106 may be geographically dispersed. In other embodiments, a machine farm 38 may be administered as a single entity. In still other embodiments, the machine farm 38 includes a plurality of machine farms 38. The servers 106 within each machine farm 38 can be heterogeneous - one or more of the servers 106 or machines 106 can operate according to one type of operating system platform (e.g., WINDOWS NT, manufactured by Microsoft Corp. of Redmond, Washington), while one or more of the other servers 106 can operate on according to another type of operating system platform (e.g., Unix, Linux, or Mac OS X).

In one embodiment, servers 106 in the machine farm 38 may be stored in high-density rack systems, along with associated storage systems, and located in an enterprise data center. In this embodiment, consolidating the servers 106 in this way may improve system manageability, data security, the physical security of the system, and system performance by locating servers 106 and high performance storage systems on localized high performance networks. Centralizing the servers 106 and storage systems and coupling them with advanced system management tools allows more efficient use of server resources.

The servers 106 of each machine farm 38 do not need to be physically proximate to another server 106 in the same machine farm 38. Thus, the group of servers 106 logically grouped as a machine farm 38 may be interconnected using a wide-area network (WAN) connection or a metropolitan-area network (MAN) connection. For example, a machine farm 38 may include servers 106 physically located in different continents or different regions of a continent, country, state, city, campus, or room. Data transmission speeds between servers 106 in the machine farm 38 can be increased if the servers 106 are connected using a local-area network (LAN) connection or some form of direct connection. Additionally, a heterogeneous machine farm 38 may include one or more servers 106 operating according to a type of operating system, while one or more other servers 106 execute one or more types of hypervisors rather than operating systems. In these embodiments, hypervisors may be used to emulate virtual hardware, partition physical hardware, virtualize physical hardware, and execute virtual machines that provide access to computing environments, allowing multiple operating systems to run concurrently on a host computer. Native hypervisors may run directly on the host computer. Hypervisors may include VMware ESX/ESXi, manufactured by VMWare, Inc., of Palo Alto, California; the Xen hypervisor, an open source product whose development is overseen by Citrix Systems, Inc.; the HYPER-V hypervisors provided by Microsoft or others. Hosted hypervisors may run within an operating system on a second software level. Examples of hosted hypervisors may include VMware Workstation and VIRTUALBOX.

Management of the machine farm 38 may be de-centralized. For example, one or more servers 106 may comprise components, subsystems and modules to support one or more management services for the machine farm 38. In one of these embodiments, one or more servers 106 provide functionality for management of dynamic data, including techniques for handling failover, data replication, and increasing the robustness of the machine farm 38. Each server 106 may communicate with a persistent store and, in some embodiments, with a dynamic store.

Server 106 may be a file server, application server, web server, proxy server, appliance, network appliance, gateway, gateway server, virtualization server, deployment server, SSL VPN server, or firewall. In one embodiment, the server 106 may be referred to as a remote machine or a node. In another embodiment, a plurality of nodes 290 may be in the path between any two communicating servers.

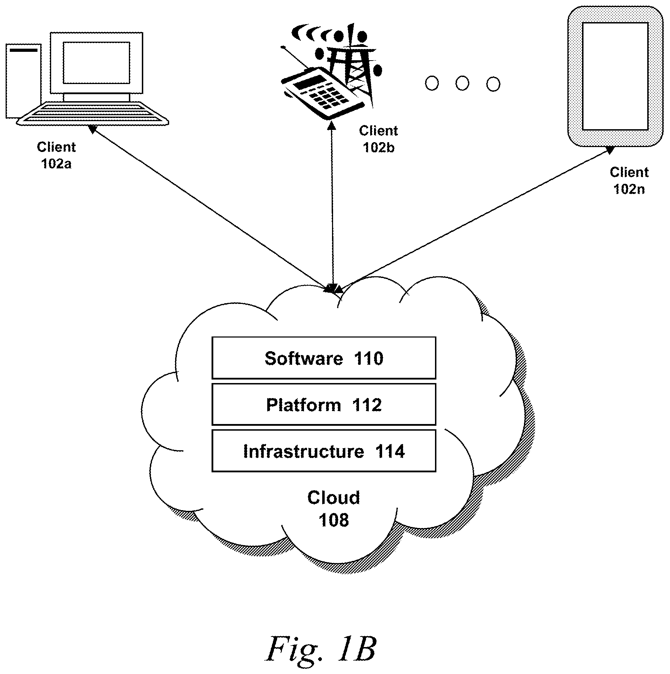

Referring to Fig. 1B, a cloud computing environment is depicted. A cloud computing environment may provide client 102 with one or more resources provided by a network environment. The cloud computing environment may include one or more clients 102a-102n, in communication with the cloud 108 over one or more networks 104. Clients 102 may include, e.g., thick clients, thin clients, and zero clients. A thick client may provide at least some functionality even when disconnected from the cloud 108 or servers 106. A thin client or a zero client may depend on the connection to the cloud 108 or server 106 to provide functionality. A zero client may depend on the cloud 108 or other networks 104 or servers 106 to retrieve operating system data for the client device. The cloud 108 may include back end platforms, e.g., servers 106, storage, server farms or data centers.

The cloud 108 may be public, private, or hybrid. Public clouds may include public servers 106 that are maintained by third parties to the clients 102 or the owners of the clients. The servers 106 may be located off-site in remote geographical locations as disclosed above or otherwise. Public clouds may be connected to the servers 106 over a public network. Private clouds may include private servers 106 that are physically maintained by clients 102 or owners of clients. Private clouds may be connected to the servers 106 over a private network 104. Hybrid clouds 108 may include both the private and public networks 104 and servers 106.

The cloud 108 may also include a cloud based delivery, e.g. Software as a Service (SaaS) 110, Platform as a Service (PaaS) 112, and Infrastructure as a Service (IaaS) 114. IaaS may refer to a user renting the use of infrastructure resources that are needed during a specified time period. IaaS providers may offer storage, networking, servers or virtualization resources from large pools, allowing the users to quickly scale up by accessing more resources as needed. Examples of IaaS can include infrastructure and services (e.g., EG-32) provided by OVH HOSTING of Montreal, Quebec, Canada, AMAZON WEB SERVICES provided by Amazon.com, Inc., of Seattle, Washington, RACKSPACE CLOUD provided by Rackspace US, Inc., of San Antonio, Texas, Google Compute Engine provided by Google Inc. of Mountain View, California, or RIGHTSCALE provided by RightScale, Inc., of Santa Barbara, California. PaaS providers may offer functionality provided by IaaS, including, e.g., storage, networking, servers or virtualization, as well as additional resources such as, e.g., the operating system, middleware, or runtime resources. Examples of PaaS include WINDOWS AZURE provided by Microsoft Corporation of Redmond, Washington, Google App Engine provided by Google Inc., and HEROKU provided by Heroku, Inc. of San Francisco, California. SaaS providers may offer the resources that PaaS provides, including storage, networking, servers, virtualization, operating system, middleware, or runtime resources. In some embodiments, SaaS providers may offer additional resources including, e.g., data and application resources. Examples of SaaS include GOOGLE APPS provided by Google Inc., SALESFORCE provided by Salesforce.com Inc. of San Francisco, California, or OFFICE 365 provided by Microsoft Corporation. Examples of SaaS may also include data storage providers, e.g. DROPBOX provided by Dropbox, Inc. of San Francisco, California, Microsoft SKYDRIVE provided by Microsoft Corporation, Google Drive provided by Google Inc., or Apple ICLOUD provided by Apple Inc. of Cupertino, California.

Clients 102 may access IaaS resources with one or more IaaS standards, including, e.g., Amazon Elastic Compute Cloud (EC2), Open Cloud Computing Interface (OCCI), Cloud Infrastructure Management Interface (CIMI), or OpenStack standards. Some IaaS standards may allow clients access to resources over HTTP, and may use Representational State Transfer (REST) protocol or Simple Object Access Protocol (SOAP). Clients 102 may access PaaS resources with different PaaS interfaces. Some PaaS interfaces use HTTP packages, standard Java APIs, JavaMail API, Java Data Objects (JDO), Java Persistence API (JPA), Python APIs, web integration APIs for different programming languages including, e.g., Rack for Ruby, WSGI for Python, or PSGI for Perl, or other APIs that may be built on REST, HTTP, XML, or other protocols. Clients 102 may access SaaS resources through the use of web-based user interfaces, provided by a web browser (e.g. GOOGLE CHROME, Microsoft INTERNET EXPLORER, or Mozilla Firefox provided by Mozilla Foundation of Mountain View, California). Clients 102 may also access SaaS resources through smartphone or tablet applications, including, e.g., Salesforce Sales Cloud, or Google Drive app. Clients 102 may also access SaaS resources through the client operating system, including, e.g., Windows file system for DROPBOX.

In some embodiments, access to IaaS, PaaS, or SaaS resources may be authenticated. For example, a server or authentication server may authenticate a user via security certificates, HTTPS, or API keys. API keys may include various encryption standards such as, e.g., Advanced Encryption Standard (AES). Data resources may be sent over Transport Layer Security (TLS) or Secure Sockets Layer (SSL).

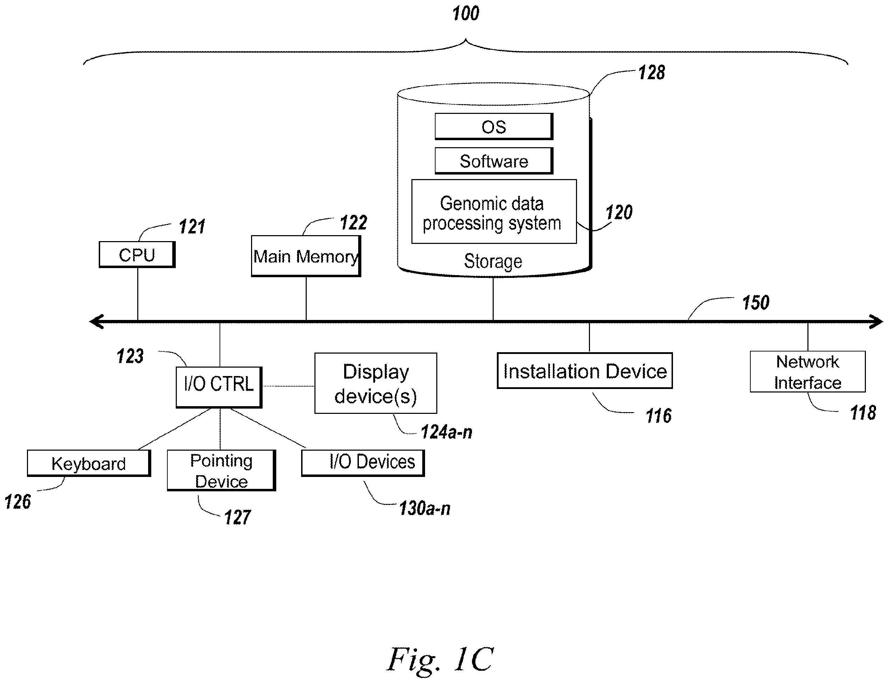

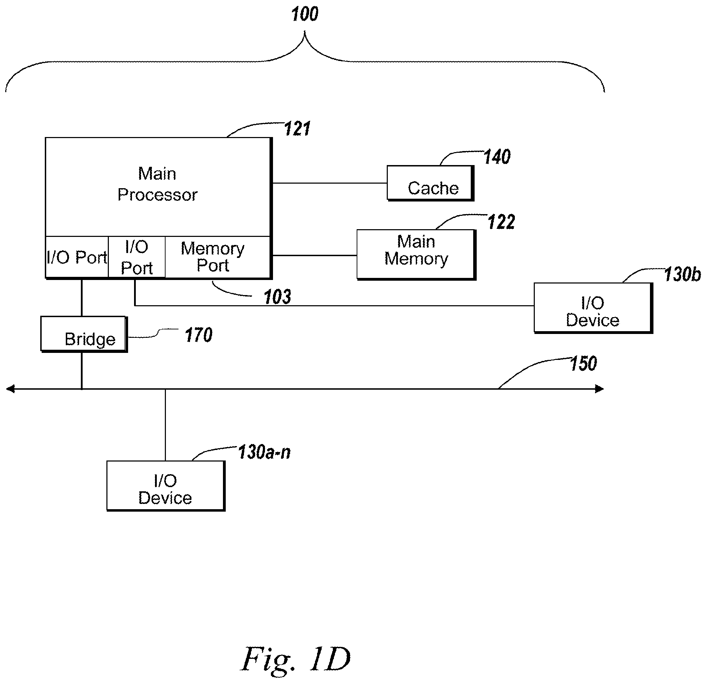

The client 102 and server 106 may be deployed as and/or executed on any type and form of computing device, e.g. a computer, network device or appliance capable of communicating on any type and form of network and performing the operations described herein. FIGs. 1C and 1D depict block diagrams of a computing device 100 useful for practicing an embodiment of the client 102 or a server 106. As shown in FIGs. 1C and 1D, each computing device 100 includes a central processing unit 121, and a main memory unit 122. As shown in FIG. 1C, a computing device 100 may include a storage device 128, an installation device 116, a network interface 118, an I/O controller 123, display devices 124a-124n, a keyboard 126 and a pointing device 127, e.g. a mouse. The storage device 128 may include, without limitation, an operating system, software, and a software of a genomic data processing system 120. As shown in FIG. 1D, each computing device 100 may also include additional optional elements, e.g. a memory port 103, a bridge 170, one or more input/output devices 130a-130n (generally referred to using reference numeral 130), and a cache memory 140 in communication with the central processing unit 121.

The central processing unit 121 is any logic circuitry that responds to and processes instructions fetched from the main memory unit 122. In many embodiments, the central processing unit 121 is provided by a microprocessor unit, e.g.: those manufactured by Intel Corporation of Mountain View, California; those manufactured by Motorola Corporation of Schaumburg, Illinois; the ARM processor and TEGRA system on a chip (SoC) manufactured by Nvidia of Santa Clara, California; the POWER7 processor, those manufactured by International Business Machines of White Plains, New York; or those manufactured by Advanced Micro Devices of Sunnyvale, California. The computing device 100 may be based on any of these processors, or any other processor capable of operating as described herein. The central processing unit 121 may utilize instruction level parallelism, thread level parallelism, different levels of cache, and multi-core processors. A multi-core processor may include two or more processing units on a single computing component. Examples of multi-core processors include the AMD PHENOM IIX2, INTEL CORE i5 and INTEL CORE i7.

Main memory unit 122 may include one or more memory chips capable of storing data and allowing any storage location to be directly accessed by the microprocessor 121. Main memory unit 122 may be volatile and faster than storage 128 memory. Main memory units 122 may be Dynamic random access memory (DRAM) or any variants, including static random access memory (SRAM), Burst SRAM or SynchBurst SRAM (BSRAM), Fast Page Mode DRAM (FPM DRAM), Enhanced DRAM (EDRAM), Extended Data Output RAM (EDO RAM), Extended Data Output DRAM (EDO DRAM), Burst Extended Data Output DRAM (BEDO DRAM), Single Data Rate Synchronous DRAM (SDR SDRAM), Double Data Rate SDRAM (DDR SDRAM), Direct Rambus DRAM (DRDRAM), or Extreme Data Rate DRAM (XDR DRAM). In some embodiments, the main memory 122 or the storage 128 may be non-volatile; e.g., non-volatile read access memory (NVRAM), flash memory non-volatile static RAM (nvSRAM), Ferroelectric RAM (FeRAM), Magnetoresistive RAM (MRAM), Phase-change memory (PRAM), conductive-bridging RAM (CBRAM), Silicon-Oxide-Nitride-Oxide-Silicon (SONOS), Resistive RAM (RRAM), Racetrack, Nano-RAM (NRAM), or Millipede memory. The main memory 122 may be based on any of the above described memory chips, or any other available memory chips capable of operating as described herein. In the embodiment shown in FIG. 1C, the processor 121 communicates with main memory 122 via a system bus 150 (described in more detail below). FIG. 1D depicts an embodiment of a computing device 100 in which the processor communicates directly with main memory 122 via a memory port 103. For example, in FIG. 1D the main memory 122 may be DRDRAM.

FIG. 1D depicts an embodiment in which the main processor 121 communicates directly with cache memory 140 via a secondary bus, sometimes referred to as a backside bus. In other embodiments, the main processor 121 communicates with cache memory 140 using the system bus 150. Cache memory 140 typically has a faster response time than main memory 122 and is typically provided by SRAM, BSRAM, or EDRAM. In the embodiment shown in FIG. 1D, the processor 121 communicates with various I/O devices 130 via a local system bus 150. Various buses may be used to connect the central processing unit 121 to any of the I/O devices 130, including a PCI bus, a PCI-X bus, or a PCI-Express bus, or a NuBus. For embodiments in which the I/O device is a video display 124, the processor 121 may use an Advanced Graphics Port (AGP) to communicate with the display 124 or the I/O controller 123 for the display 124. FIG. 1D depicts an embodiment of a computer 100 in which the main processor 121 communicates directly with I/O device 130b or other processors 121' via HYPERTRANSPORT, RAPIDIO, or INFINIBAND communications technology. FIG. 1D also depicts an embodiment in which local busses and direct communication are mixed: the processor 121 communicates with I/O device 130a using a local interconnect bus while communicating with I/O device 130b directly.

A wide variety of I/O devices 130a-130n may be present in the computing device 100. Input devices may include keyboards, mice, trackpads, trackballs, touchpads, touch mice, multi-touch touchpads and touch mice, microphones, multi-array microphones, drawing tablets, cameras, single-lens reflex camera (SLR), digital SLR (DSLR), CMOS sensors, accelerometers, infrared optical sensors, pressure sensors, magnetometer sensors, angular rate sensors, depth sensors, proximity sensors, ambient light sensors, gyroscopic sensors, or other sensors. Output devices may include video displays, graphical displays, speakers, headphones, inkjet printers, laser printers, and 3D printers.

Devices 130a-130n may include a combination of multiple input or output devices, including, e.g., Microsoft KINECT, Nintendo Wiimote for the WII, Nintendo WII U GAMEPAD, or Apple IPHONE. Some devices 130a-130n allow gesture recognition inputs through combining some of the inputs and outputs. Some devices 130a-130n provides for facial recognition which may be utilized as an input for different purposes including authentication and other commands. Some devices 130a-130n provides for voice recognition and inputs, including, e.g., Microsoft KINECT, SIRI for IPHONE by Apple, Google Now or Google Voice Search.

Additional devices 130a-130n have both input and output capabilities, including, e.g., haptic feedback devices, touchscreen displays, or multi-touch displays. Touchscreen, multi-touch displays, touchpads, touch mice, or other touch sensing devices may use different technologies to sense touch, including, e.g., capacitive, surface capacitive, projected capacitive touch (PCT), in-cell capacitive, resistive, infrared, waveguide, dispersive signal touch (DST), in-cell optical, surface acoustic wave (SAW), bending wave touch (BWT), or force-based sensing technologies. Some multi-touch devices may allow two or more contact points with the surface, allowing advanced functionality including, e.g., pinch, spread, rotate, scroll, or other gestures. Some touchscreen devices, including, e.g., Microsoft PIXELSENSE or Multi-Touch Collaboration Wall, may have larger surfaces, such as on a table-top or on a wall, and may also interact with other electronic devices. Some I/O devices 130a-130n, display devices 124a-124n or group of devices may be augment reality devices. The I/O devices may be controlled by an I/O controller 123 as shown in FIG. 1C. The I/O controller may control one or more I/O devices, such as, e.g., a keyboard 126 and a pointing device 127, e.g., a mouse or optical pen. Furthermore, an I/O device may also provide storage and/or an installation medium 116 for the computing device 100. In still other embodiments, the computing device 100 may provide USB connections (not shown) to receive handheld USB storage devices. In further embodiments, an I/O device 130 may be a bridge between the system bus 150 and an external communication bus, e.g. a USB bus, a SCSI bus, a FireWire bus, an Ethernet bus, a Gigabit Ethernet bus, a Fibre Channel bus, or a Thunderbolt bus.

In some embodiments, display devices 124a-124n may be connected to I/O controller 123. Display devices may include, e.g., liquid crystal displays (LCD), thin film transistor LCD (TFT-LCD), blue phase LCD, electronic papers (e-ink) displays, flexile displays, light emitting diode displays (LED), digital light processing (DLP) displays, liquid crystal on silicon (LCOS) displays, organic light-emitting diode (OLED) displays, active-matrix organic light-emitting diode (AMOLED) displays, liquid crystal laser displays, time-multiplexed optical shutter (TMOS) displays, or 3D displays. Examples of 3D displays may use, e.g. stereoscopy, polarization filters, active shutters, or autostereoscopy. Display devices 124a-124n may also be a head-mounted display (HMD). In some embodiments, display devices 124a-124n or the corresponding I/O controllers 123 may be controlled through or have hardware support for OPENGL or DIRECTX API or other graphics libraries.

In some embodiments, the computing device 100 may include or connect to multiple display devices 124a-124n, which each may be of the same or different type and/or form. As such, any of the I/O devices 130a-130n and/or the I/O controller 123 may include any type and/or form of suitable hardware, software, or combination of hardware and software to support, enable or provide for the connection and use of multiple display devices 124a-124n by the computing device 100. For example, the computing device 100 may include any type and/or form of video adapter, video card, driver, and/or library to interface, communicate, connect or otherwise use the display devices 124a-124n. In one embodiment, a video adapter may include multiple connectors to interface to multiple display devices 124a-124n. In other embodiments, the computing device 100 may include multiple video adapters, with each video adapter connected to one or more of the display devices 124a-124n. In some embodiments, any portion of the operating system of the computing device 100 may be configured for using multiple displays 124a-124n. In other embodiments, one or more of the display devices 124a-124n may be provided by one or more other computing devices 100a or 100b connected to the computing device 100, via the network 104. In some embodiments software may be designed and constructed to use another computer's display device as a second display device 124a for the computing device 100. For example, in one embodiment, an Apple iPad may connect to a computing device 100 and use the display of the device 100 as an additional display screen that may be used as an extended desktop. One ordinarily skilled in the art will recognize and appreciate the various ways and embodiments that a computing device 100 may be configured to have multiple display devices 124a-124n.

Referring again to FIG. 1C, the computing device 100 may comprise a storage device 128 (e.g. one or more hard disk drives or redundant arrays of independent disks) for storing an operating system or other related software, and for storing application software programs such as any program related to the software for the genomic data processing system 120. Examples of storage device 128 include, e.g., hard disk drive (HDD); optical drive including CD drive, DVD drive, or BLU-RAY drive; solid-state drive (SSD); USB flash drive; or any other device suitable for storing data. Some storage devices may include multiple volatile and non-volatile memories, including, e.g., solid state hybrid drives that combine hard disks with solid state cache. Some storage device 128 may be non-volatile, mutable, or read-only. Some storage device 128 may be internal and connect to the computing device 100 via a bus 150. Some storage devices 128 may be external and connect to the computing device 100 via an I/O device 130 that provides an external bus. Some storage device 128 may connect to the computing device 100 via the network interface 118 over a network 104, including, e.g., the Remote Disk for MACBOOK AIR by Apple. Some client devices 100 may not require a non-volatile storage device 128 and may be thin clients or zero clients 102. Some storage device 128 may also be used as an installation device 116, and may be suitable for installing software and programs. Additionally, the operating system and the software can be run from a bootable medium, for example, a bootable CD, e.g. KNOPPIX, a bootable CD for GNU/Linux that is available as a GNU/Linux distribution from knoppix.net.

Client device 100 may also install software or application from an application distribution platform. Examples of application distribution platforms include the App Store for iOS provided by Apple, Inc., the Mac App Store provided by Apple, Inc., GOOGLE PLAY for Android OS provided by Google Inc., Chrome Webstore for CHROME OS provided by Google Inc., and Amazon Appstore for Android OS and KINDLE FIRE provided by Amazon.com, Inc. An application distribution platform may facilitate installation of software on a client device 102. An application distribution platform may include a repository of applications on a server 106 or a cloud 108, which the clients 102a-102n may access over a network 104. An application distribution platform may include application developed and provided by various developers. A user of a client device 102 may select, purchase and/or download an application via the application distribution platform.

Furthermore, the computing device 100 may include a network interface 118 to interface to the network 104 through a variety of connections including, but not limited to, standard telephone lines LAN or WAN links (*e.g.,* 802.11, T1, T3, Gigabit Ethernet, Infiniband), broadband connections (*e.g.*, ISDN, Frame Relay, ATM, Gigabit Ethernet, Ethernet-over-SONET, ADSL, VDSL, BPON, GPON, fiber optical including FiOS), wireless connections, or some combination of any or all of the above. Connections can be established using a variety of communication protocols (e.g., TCP/IP, Ethernet, ARCNET, SONET, SDH, Fiber Distributed Data Interface (FDDI), IEEE 802.11a/b/g/n/ac CDMA, GSM, WiMax and direct asynchronous connections). In one embodiment, the computing device 100 communicates with other computing devices 100' via any type and/or form of gateway or tunneling protocol e.g. Secure Socket Layer (SSL) or Transport Layer Security (TLS), or the Citrix Gateway Protocol manufactured by Citrix Systems, Inc. of Ft. Lauderdale, Florida. The network interface 118 may comprise a built-in network adapter, network interface card, PCMCIA network card, EXPRESSCARD network card, card bus network adapter, wireless network adapter, USB network adapter, modem or any other device suitable for interfacing the computing device 100 to any type of network capable of communication and performing the operations described herein.

A computing device 100 of the sort depicted in FIGs. 1B and 1C may operate under the control of an operating system, which controls scheduling of tasks and access to system resources. The computing device 100 can be running any operating system such as any of the versions of the MICROSOFT WINDOWS operating systems, the different releases of the Unix and Linux operating systems, any version of the MAC OS for Macintosh computers, any embedded operating system, any real-time operating system, any open source operating system, any proprietary operating system, any operating systems for mobile computing devices, or any other operating system capable of running on the computing device and performing the operations described herein. Typical operating systems include, but are not limited to: WINDOWS 2000, WINDOWS Server 2022, WINDOWS CE, WINDOWS Phone, WINDOWS XP, WINDOWS VISTA, and WINDOWS 7, WINDOWS RT, and WINDOWS 8 all of which are manufactured by Microsoft Corporation of Redmond, Washington; MAC OS and iOS, manufactured by Apple, Inc. of Cupertino, California; and Linux, a freely-available operating system, e.g. Linux Mint distribution ("distro") or Ubuntu, distributed by Canonical Ltd. of London, United Kingdom; or Unix or other Unix-like derivative operating systems; and Android, designed by Google, of Mountain View, California, among others. Some operating systems, including, e.g., the CHROME OS by Google, may be used on zero clients or thin clients, including, e.g., CHROMEBOOKS.

The computer system 100 can be any workstation, telephone, desktop computer, laptop or notebook computer, netbook, ULTRABOOK, tablet, server, handheld computer, mobile telephone, smartphone or other portable telecommunications device, media playing device, a gaming system, mobile computing device, or any other type and/or form of computing, telecommunications or media device that is capable of communication. The computer system 100 has sufficient processor power and memory capacity to perform the operations described herein. In some embodiments, the computing device 100 may have different processors, operating systems, and input devices consistent with the device. The Samsung GALAXY smartphones, e.g., operate under the control of Android operating system developed by Google, Inc. GALAXY smartphones receive input via a touch interface.

In some embodiments, the computing device 100 is a gaming system. For example, the computer system 100 may comprise a PLAYSTATION 3, or PERSONAL PLAYSTATION PORTABLE (PSP), or a PLAYSTATION VITA device manufactured by the Sony Corporation of Tokyo, Japan, a NINTENDO DS, NINTENDO 3DS, NINTENDO WII, or a NINTENDO WII U device manufactured by Nintendo Co., Ltd., of Kyoto, Japan, an XBOX 360 device manufactured by the Microsoft Corporation of Redmond, Washington.

In some embodiments, the computing device 100 is a digital audio player such as the Apple IPOD, IPOD Touch, and IPOD NANO lines of devices, manufactured by Apple Computer of Cupertino, California. Some digital audio players may have other functionality, including, e.g., a gaming system or any functionality made available by an application from a digital application distribution platform. For example, the IPOD Touch may access the Apple App Store. In some embodiments, the computing device 100 is a portable media player or digital audio player supporting file formats including, but not limited to, MP3, WAV, M4A/AAC, WMA Protected AAC, AIFF, Audible audiobook, Apple Lossless audio file formats and .mov, .m4v, and .mp4 MPEG-4 (H.264/MPEG-4 AVC) video file formats.

In some embodiments, the computing device 100 is a tablet e.g. the IPAD line of devices by Apple; GALAXY TAB family of devices by Samsung; or KINDLE FIRE, by Amazon.com, Inc. of Seattle, Washington. In other embodiments, the computing device 100 is an eBook reader, e.g. the KINDLE family of devices by Amazon.com, or NOOK family of devices by Barnes & Noble, Inc. of New York City, New York.

In some embodiments, the communications device 102 includes a combination of devices, e.g. a smartphone combined with a digital audio player or portable media player. For example, one of these embodiments is a smartphone, e.g. the IPHONE family of smartphones manufactured by Apple, Inc.; a Samsung GALAXY family of smartphones manufactured by Samsung, Inc.; or a Motorola DROID family of smartphones. In yet another embodiment, the communications device 102 is a laptop or desktop computer equipped with a web browser and a microphone and speaker system, e.g. a telephony headset. In these embodiments, the communications devices 102 are web-enabled and can receive and initiate phone calls. In some embodiments, a laptop or desktop computer is also equipped with a webcam or other video capture device that enables video chat and video call.

In some embodiments, the status of one or more machines 102, 106 in the network 104 are monitored, generally as part of network management. In one of these embodiments, the status of a machine may include an identification of load information (e.g., the number of processes on the machine, CPU and memory utilization), of port information (e.g., the number of available communication ports and the port addresses), or of session status (e.g., the duration and type of processes, and whether a process is active or idle). In another of these embodiments, this information may be identified by a plurality of metrics, and the plurality of metrics can be applied at least in part towards decisions in load distribution, network traffic management, and network failure recovery as well as any aspects of operations of the present solution described herein. Aspects of the operating environments and components described above will become apparent in the context of the systems and methods disclosed herein.

### B. Computer complemented method for identifying mutations in cell-free DNA

cfDNA encompasses all small DNA fragments (~167 base pairs) circulating in the blood, which can be isolated from the plasma component. In cancer subjects, some of these fragments come from cancer cells (i.e., circulating tumor DNA, or ctDNA), providing a window into the somatic, or acquired, mutations in their tumor(s).

Somatic mutation calling differs from germline mutation calling in that the fraction of DNA molecules harboring a mutation can vary widely due to tumor heterogeneity and chromosomal gains and losses. This challenge is compounded when trying to identify tumor mutations in cfDNA, as the fraction of tumor-derived DNA can be extremely low (~0.1%). Consequently, the mutation fractions in cfDNA are often lower than those observed in tissue samples from the same subject and may approach the noise levels of next-generation sequencing workflows. This can make it impossible to distinguish true somatic mutations from artifacts. Effective somatic mutation calling from cfDNA, particularly for early-stage cancer subjects, requires suppressing errors introduced in sample preparation and sequencing.

One technique that has been developed for error suppression is 'unique molecular indexing' (UMIs), also known as molecular barcoding. Each DNA molecule is tagged with sequence adapters containing a specific sequence barcode (a UMI) to distinguish it from other molecules. As part of sample preparation, each molecule is copied multiple times, and each copy contains the same UMI. The techniques and methods discussed below identify all the copies of each molecule, group them together, and collapse them to derive a single consensus without sequencing errors. Further, the consensus mutations are compared with consensus mutations identified in WBC sequence reads of the same subject. Any germline variants appearing in the consensus mutations associated with the cfDNA sequence reads can be removed, thereby providing an accurate list of identified hematopoietic variants. This reduces the errors associated with identification of mutations in cfDNA sequence reads. The reduction in error improves the accuracy and the confidence of the identified mutations in the cfDNA.

Assay design and workflow for identification of mutations or variants in the cfDNA sequence reads is discussed below.

### Assay Design

Sequence-specific DNA probes can be used to capture the desired regions of the genome for cfDNA analysis. As one application of cfDNA analysis is to detect the presence of tumor-derived DNA, the probability that a given cancer would have at least one mutation detectable by the assay has been improved.

Data from more than 20,000 tumors can be leveraged to select the most frequently mutated and the most clinically relevant protein-coding exons according to the following criteria.
1. Exons with at least one OncoKB Level 1-4 mutation in MSK-IMPACT 20k. (OncoKB is a knowledgebase of the biological and clinical effects of tumor mutations, published in PMID 28890946. 'MSK-IMPACT 20k' refers to the first 20,000 tumors sequenced using the MSK-IMPACT platform.)
2. Exons with at least 10 mutations at hotspot sites in MSK-IMAPCT 20k. (The list of hotspots is published in PMID 29247016.)
3. Exons with >30 mutations per Megabase in MSK-IMPACT 20k.
4. All exons in protein kinase domains of selected druggable kinase genes (n=21).
5. All exons in frequently mutated tumor suppressor genes (n=25).
6. Additional exons and genes based on expert selection.
7. >160 microsatellite regions to detect the signature of microsatellite instability ('MSI').

Altogether, these exons can cover ~230,000 base pairs and encompass part of 129 genes. Of the >20,000 subjects sequenced by MSK-IMPACT, 84% of cases have at least one mutation covered by this panel (including 94% of all breast cancers and 96% of all lung cancers).

While the above regions were included for the purpose of detecting somatic mutations with high sensitivity, probes have been designed for additional regions to detect other classes of genomic alterations, including:
1. Introns to detect structural variants that produce actionable gene fusions (in ALK, BRAF, EGFR, ETV6, FGFR2, FGFR3, MET, NTRK1, NTRK3, RET, ROS1).
2. Genes associated with clonal hematopoiesis to detect acquired mutations in blood cells.
3. >590 common SNPs to enable the characterization of genome-wide copy number profiles, identify changes in zygosity and copy number in key genes, and perform quality control (genetic fingerprinting and contamination detection).

These probes add another ~171,000 base pairs. Because the regions in this second category do not require the same ultra-high level of coverage for error suppression and mutation calling, the capture probes have been mixed in unequal ratios. This allows sequencing to provide different levels of coverage and distribute sequence reads (and costs) efficiently.

### Workflow

The workflow includes a wet lab process and a data processing process. The wet lab process includes collecting blood or body fluids (including, but not limited to, serum, plasma, sweat, tears, urine, saliva, synovial fluid, lymphatic fluid, ascites fluid, amniotic fluid, or interstitial fluid) from a cancer subject. Additionally or alternatively, in some embodiments, the subject suffers from or is at risk for ovarian cancer, breast cancer, colorectal cancer, lung cancer, prostate cancer, gastric cancer, pancreatic cancer, cervical cancer, liver cancer, bladder cancer, cancer of the urinary tract, thyroid cancer, renal cancer, carcinoma, melanoma, head and neck cancer, or brain cancer. The blood or bodily fluids can be processed to extract cfDNA using any method known in the art. For example, the blood of the subject can be subjected to 2-spin centrifugation to isolate plasma and leukocytes (or white blood cells (WBC)). CfDNA is extracted from the non-cellular portion of the centrifuged body fluid. In addition, WBC DNA is extracted from the white blood cells. In instances where the cfDNA is extracted from non-blood body fluids, the WBC DNA can be extracted from a separate blood draw from the subject. The cfDNA and the WBC DNA are input to an assay. DNA adapters containing unique molecular indexes (UMIs) can be ligated or attached to the ends of the cfDNA and the WBC DNA.

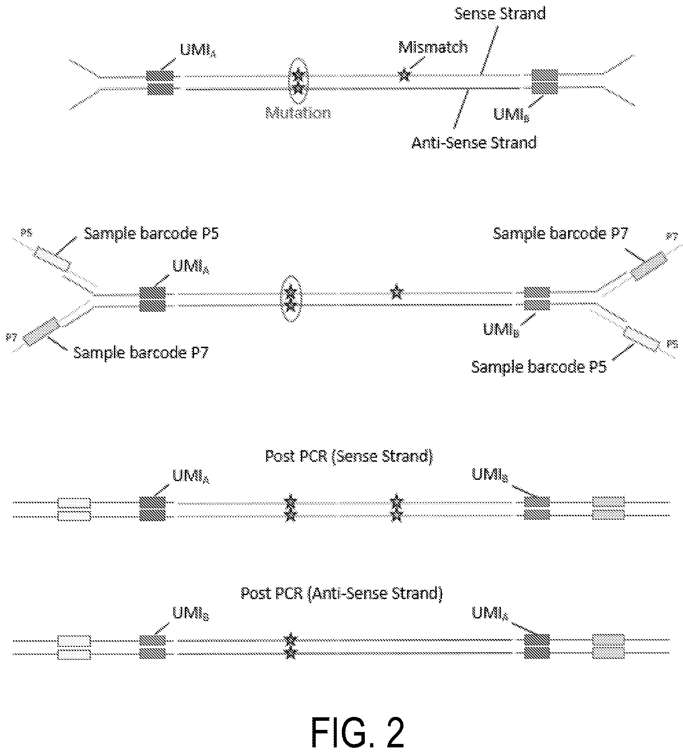

FIG. 2 illustrates cfDNA strands with attached duplex UMIs and sample barcodes. In particular, FIG. 2 shows a sense strand and an anti-sense strand of a double stranded cfDNA. Each of the strands of the cfDNA include UMIs attached at each end. For example, the sense strand has UMI A on one end (5' or forward end) and UMI B on the opposing end (3' or reverse end), while the anti-sense strand has UMI A' on one end (3' or reverse end) and UMI B' on the other end (5' or forward end). UMI A' is complementary to UMI A, while UMI B' is complementary to UMI B. DNA adapters containing these UMIs can be ligated or attached to the ends of the cfDNA sense and anti-sense strands. In one or more embodiments, the DNA adapters can include, but not limited to, those provided by Integrated DNA Technologies (IDT). The ligated cfDNA is amplified using polymerase chain reaction (PCR) techniques. However, unique dual-indexes are added to the ligated cfDNA during the PCR process. For example, the sense strand includes the sample barcode P5 adjacent to the UMI A at the forward end and the sample barcode P7 adjacent to the UMI B at the reverse end. Similarly, the anti-sense strand includes the sample barcode P5 adjacent to the UMI B' at the forward end and the sample barcode P5 adjacent to the UMI A' at the reverse end. In one or more embodiments, the PCR process can utilize index primers provided by IDT. The PCR process can generate copies of each of the sense strand and the anti-sense strand including the respective UMIs and the sample barcodes. WBC DNA molecules can optionally be similarly barcoded. For example, the UMIs can be ligated or attached to the forward and reverse ends of the sense and anti-sense strands of the WBC DNAs. In addition, PCR techniques can be used to include sample barcodes on each end of the WBC DNAs. In one or more embodiments, the sample barcodes include at least one PCR primer binding site, at least one sequencing primer binding site, or any combination thereof. In one or more embodiments, the sample barcode sequence comprises 2-20 nucleotides.

cfDNAs and WBC DNAs associated with the same subject can be assigned unique sample barcodes. In this manner, subject specific analysis of the cfDNA and WBC DNA can be carried out. The process of adding sample barcodes to the cfDNA and the WBC DNA is known as multiplexing. This allows large numbers of libraries to be pooled and sequenced simultaneously during a single sequencing run. With multiplexed libraries, unique sample barcode sequences (see *e.g.,* FIG. 2) are incorporated *via* PCR to each DNA molecule during library preparation so that each sequence read can be identified and sorted. Sequencing reads are then sorted according to their sample barcodes (*i.e.,* the sequence reads are assigned to a given subject sample) using a computational process called de-multiplexing, allowing for proper alignment. However, such multiplex approaches come with a risk of sample misidentification due to sample barcode mis-assignment, according to Kircher M *et al., Nucleic Acids Res.* 2513-2524 (2012). Incorrect assignment of sequencing reads may lead to misalignment of reads or incorrect assumptions in downstream analysis. Possible causes for incorrect sample barcode assignment are sample barcode contamination, sample barcode hopping during PCR or NGS.

Many next generation sequencing-based techniques rely upon a PCR amplification step to increase the concentration of the library generated from the DNA sample prior to next-generation sequencing. Following alignment to the genome, PCR duplicates are generally identified and removed as there are inherent biases in the amplification step as some sequences become overrepresented in the final library compared to their actual abundance within the DNA sample obtained from a subject. In some next generation sequencing-based techniques, the Picard software (Broad Institute, Cambridge MA) is used to identify and remove PCR duplicates using their genomic coordinates.

The PCR copies of the cfDNA and the WBC DNA can be used, as discussed below, for error suppression to produce highly accurate consensus sequences. The PCR copies can be provided to a next-generation (NG) sequencing device such as, for example, an Illumina sequencer, a Lymphotrac sequencer, an Ion Torrent sequencer, and a 454 pyro-sequencer. The NG sequencer can provide detailed chromosome analysis, and can employ techniques such as array comparative genomic hybridization (CGH), microarray, oligo array, single nucleotide polymorphism (SNP) array, whole genome array (WGA), and the like. The NG sequencer can provide raw genomic data to a genomic data processing system (such as the genomic data processing system 120, FIG. 1C). In particular, the NG sequencer can provide genomic data derived from biological samples including copies of the cfDNA and the WBC DNA associated with one or more subjects.

Somatic allele fractions in cfDNA are often lower than those observed in tissue samples. Accurate somatic mutation calling at very low allele fractions (<0.1%) is challenging due to noise inherent in sample preparation procedures and Next Generation Sequencing. The techniques discussed herein can reduce noise levels below desired mutation detection levels.

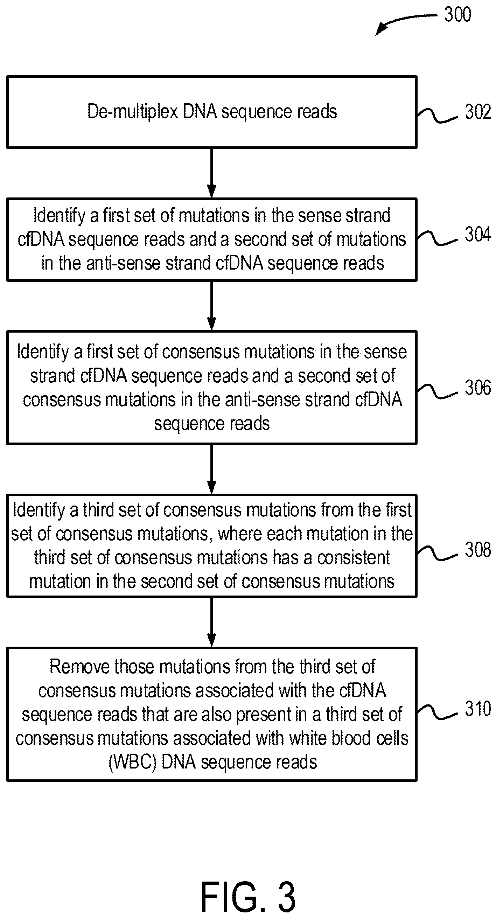

FIG. 3 illustrates a flow diagram of a mutation identification process 300. In particular, the mutation identification process 300 can be executed by the genomic data processing system 120 shown in FIG. 1C. The genomic data processing system can include or execute on one or more processors and can include scripts, modules, or computer-executable code, which when executed by one or more processors, can cause the genomic data processing system 120 to perform the process 300. The process 300 includes de-multiplexing the DNA sequence reads received from the NGS (302). De-multiplexing the DNA sequence reads can include sorting the sequence reads to their respective samples (or unique identity). By using both sample barcode and UMIs, errors that may arise due to index-hopping can be reduced. The de-multiplexing of the DNA sequence reads can be applied to both the cfDNA sequence reads and the WBC DNA sequence reads, resulting in sorted cfDNA sequence reads associated with the same sample barcodes as well as sorted WBC DNAs sequence reads associated with the same sample barcodes. The cfDNA sequence reads include the cfDNA sequence reads associated with the sense strand and cfDNA sequence reads associated with the anti-sense strands. Similarly, the WBC DNA sequence reads can include both sense strand and anti-sense strand sequence reads.

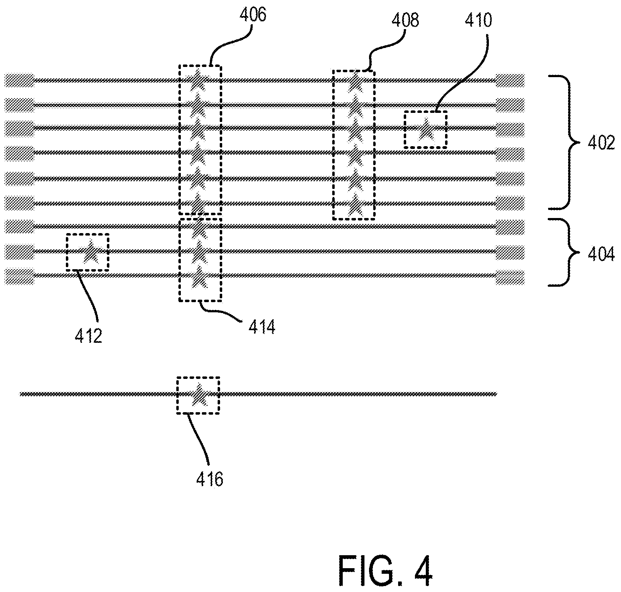

The process 300 further includes identifying a first set of mutations in the sense strand cfDNA sequence reads and identifying a second set of mutations in the anti-sense strand cfDNA sequence reads (304). FIG. 4 illustrates example sense strand cfDNA sequence reads 402 and anti-sense strand cfDNA reads 404. Mutations 406, 408, and 410 can be identified in the sense strand cfDNA sequence reads, while mutations 412 and 414 can be identified in the anti-sense strand cfDNA sequence reads. In one embodiment, the mutations can be identified by comparing the sequence reads to known mutations, for example using hotspots and genotyping. In some other embodiments, the mutations can be new mutations, and can be identified by comparing the sequence strands to the human genome database. The process 300 also can include similarly identifying mutations in the sense strand and anti-sense strand WBC DNA sequence reads. In some embodiments, the method further comprises trimming the forward and reverse UMIs from the sense strand cfDNA sequence reads and the anti-sense strand cfDNA sequence reads, and /or the sense strand WBC DNA sequence reads and the anti-sense strand WBC DNA sequence reads prior to identifying the first set of mutations and the second set of mutations.

The process 300 further includes identifying a first set of consensus mutations in the sense strand cfDNA sequence reads and a second set of consensus mutations in the anti-sense strand cfDNA sequence reads (306). The first set of consensus mutations include mutations from the first set of mutations that appear in the same position in the respective cfDNA sequence reads of sense cfDNA sequence reads. Similarly, the second set of consensus mutations include mutations from the second set of mutations that appear in the same position in the respective cfDNA sequence reads of the anti-sense cfDNA sequence reads. For example, FIG. 4 shows a first set of consensus mutations that include mutations 406 and mutations 408 in the sense strand cfDNA sequence reads 402, and a second set of consensus mutations that include the mutations 414 in the anti-sense strand cfDNA sequence reads 404. The process 300 also can include similarly identifying a first set and a second set of consensus mutations in the WBC DNA sequence reads. Identifying the first set of consensus mutations and the second set of consensus mutations can be based on several factors such as total number of sense or anti-sense sequence reads, percentage of sequence reads including the mutations, tolerance level of mutation mismatches among the sequence reads, base quality and mapping quality thresholds, and duplex versus single strand sequence reads.

The process 300 further includes identifying a third set of consensus mutations from the first set of consensus mutations, where each mutation in the third set of consensus mutations have a consistent mutation in the second set of consensus mutations (308). For example, FIG. 4 shows a third set of consensus mutations 416 includes mutations 406 form the first set of consensus mutations, as the mutations 406 have corresponding consistent mutations 414 in the second set of consensus mutations. Mutations 408 are not included in the third set as there are no corresponding consistent consensus mutations in the anti-sense cfDNA sequence reads. Consistent consensus mutations include those mutations that are complementary to each other. E.g., consensus mutation ATGC and TACG are consistent with, and complementary to, each other. In some embodiments, the process 300 may include similarly identifying a third set of consensus mutations in the WBC DNA sequence reads. Alternatively, the process does not include identifying a third set of consensus mutations in the WBC DNA sequence reads.

The process 300 further includes removing those mutations from the third set of consensus mutations associated with the cfDNA sequence reads that are also present in the WBC DNA sequence reads (*e.g.,* third set of consensus mutations associated with the WBC DNA sequence reads) (310). For example, by removing the mutations in the third set of consensus mutations in the cfDNA sequence reads that are also present in the WBC DNA sequence reads, one can remove germline variants and identify clonal hematopoietic variants. After removal, the resulting set of mutations provides a more accurate list of cancer-derived mutations present in the cfDNA of the subject, thereby improving the accuracy of detection of disease in the subject. In some embodiments, the WBC DNA will not necessarily go through the same collapsing process as the cfDNA. Error suppression isn't as critical for the control WBC DNA since the errors do not lead to false positive mutation calls. In some embodiments, the process can sequence the WBC DNA to standard (not ultra-high) depth and can still use it to filter the cfDNA data.

In one or more embodiments, the process 300 also can include a polishing step, in which a large set of normal (non-cancer) cfDNA samples is sequenced using molecular barcoding and an error distribution is created from the artifacts observed in those samples at each genomic position. This allows attachment of a confidence value to the somatic mutations called in the cfDNA sequence reads. For example, cfDNA sequence reads from normal healthy donors (e.g., at least 10 individuals, equal distribution of gender) can be analyzed with the same assay to establish background error rates. These confidence intervals associated with the mutations can be further used to determine whether a mutation or a consensus mutation is a valid mutation or an artifact. The polishing step can further improve the accuracy of detecting mutations in the cfDNA sequence reads of the subject.

The process 300 also can include utilizing blacklists to further modify the final set of mutations identified in the cfDNA sequence reads. For example, recurrent errors seen in an *n* number (e.g., 2) or more normal healthy donor cfDNA sequence reads can be added to a blacklist. Mutations appearing in the final set of mutations associated with the cfDNA sequence reads of the subject if also appear in the blacklist can be removed from the final set, thereby further improving the accuracy of detecting mutations in the cfDNA sequence reads of the subject. The process 300 may also include removing mutations from the final set of mutations based on position-specific and class-specific error models.

In one or more embodiments, at least one identified mutation discussed above is in an exon of a cancer-related gene selected from the group consisting of:
AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3, GNA11, GNAQ, GNAS, H3F3A, HIST1H3B, HRAS, IDH1, IDH2, IKZF1, INPPL1, JAK 1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL, and XPO1.

In one or more embodiments, at least one identified mutation discussed above is in an intron of a cancer-related gene selected from the group consisting of: ALK, BRAF, EGFR. ETV6, FGFR2, FGFR3, MET, NTRK1, RET and ROS1 or a promoter region of TERT. In one or more embodiments, at least one mutation identified is in a microsatellite locus for microsatellite instability. In one or more embodiments, at least one mutation identified is in cancer-related gene selected from the group consisting of: BRCA1/2, MLH1, MSH2, MSH6, PMS2. In one or more embodiments, at least one mutation identified is a deletion, an insertion, a translocation, an inversion, a copy number variant, or a point mutation.

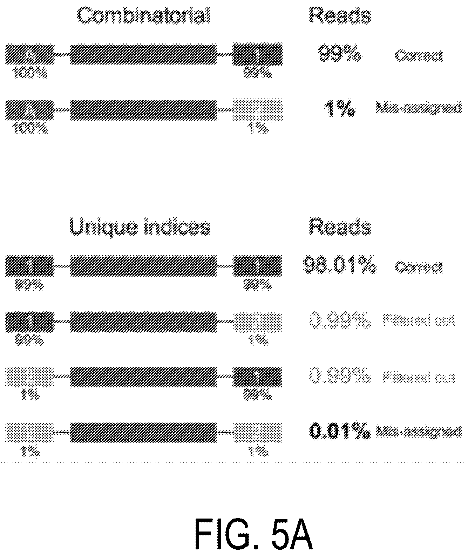

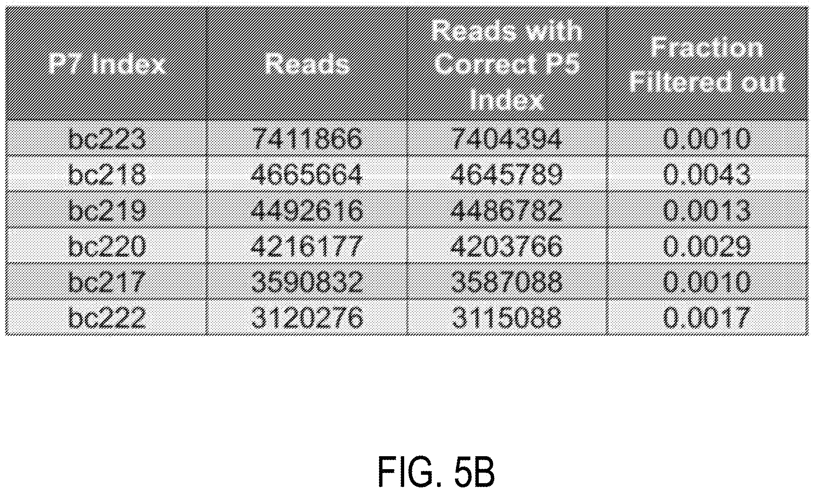

The methods of the present disclosure include the use of dual index primers, which can significantly reduce the number of incorrectly assigned reads. See FIGs. 5A and 5B. In some embodiments of the methods disclosed herein, the quality control metrics of the cfDNA/WBC DNA sequence reads are computed. Additionally, or alternatively, in some embodiments, the QC metrics for the consensus mutations are computed. QC metrics may include coverage (total or collapsed), noise level, family size distribution, and family types (dual-indexed reads, single indexed reads or singleton reads).

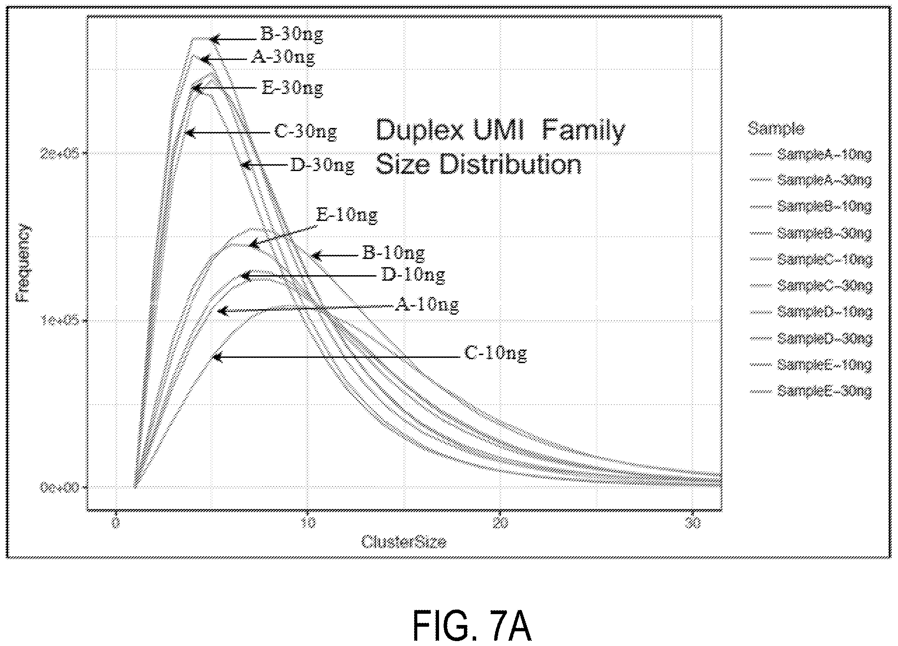

FIG. 4 represents a read family (collection of read pairs that all have the same UMI and were all derived from the same original double-stranded DNA template). This is a 'duplex' family because reads from both the sense and antisense strand of the original double-stranded DNA template are represented. It is also possible that a read family might only contain reads from one of the two strands (a 'simplex' or 'single-strand' read family). In practice, a simplex read family consists of 3 or more reads. (A family with exactly 2 reads from the same strand is 'sub-simplex'. A family with exactly 1 read is called a 'singleton'). The processes and methods discussed herein (Marianas software) performs this 'collapsing' of UMI-based read families and defines the read families as either 'duplex', 'simplex', 'sub-simplex', or 'singleton'. FIGs. 7A-7C show exemplary QC metrics from UMI-based read families.

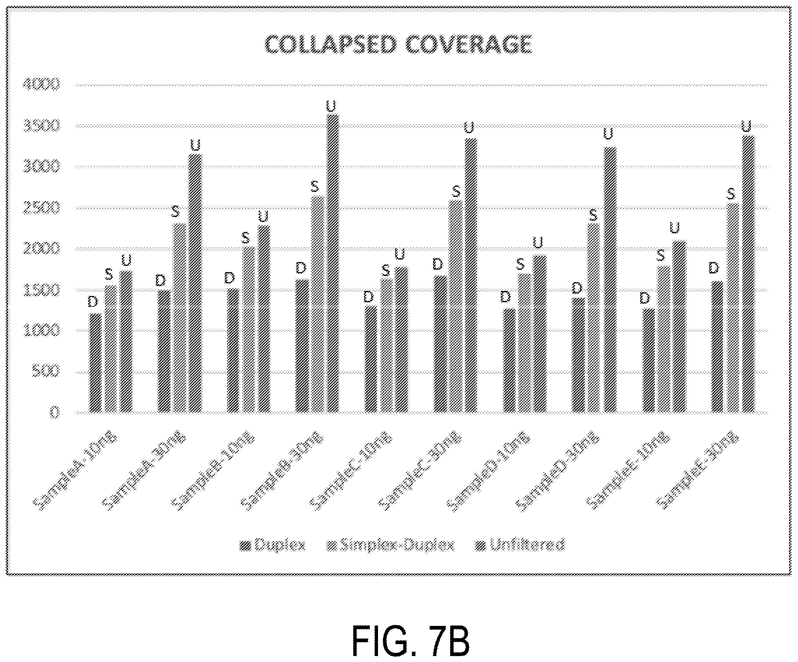

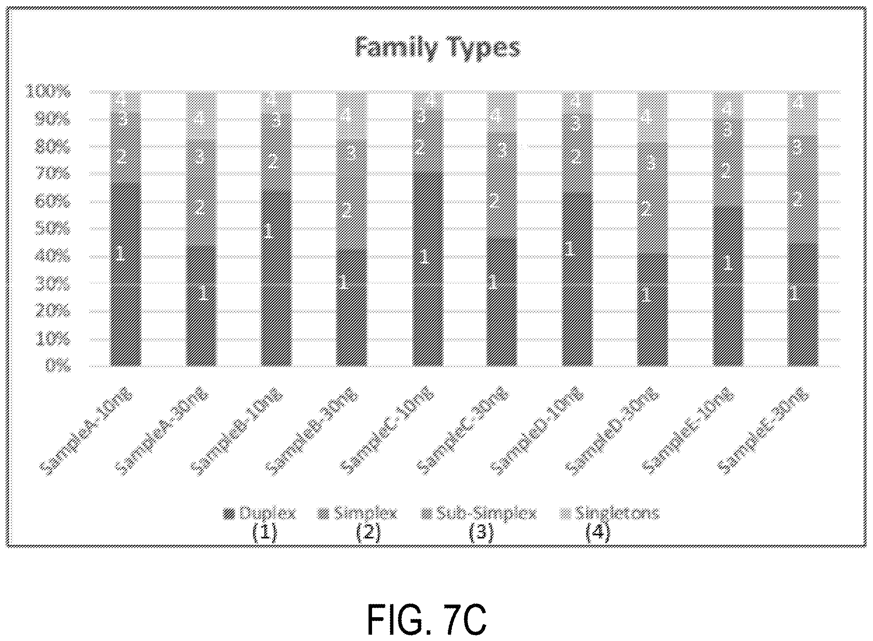

FIG. 7B illustrates an example of the collapsed coverage of UMI-based read families observed when using the data processing methods of the present disclosure. FIG. 7A illustrates an example of the family size distribution of UMI-based read families observed when using the data processing methods of the present disclosure. FIG. 7C shows an example of the fractions of various family types (dual-indexed, single indexed or singleton) of UMI-based read families observed when using the data processing methods of the present disclosure. As shown in FIG. 7C, a higher fraction of duplex read families was observed in the 10 ng cfDNA samples relative to that observed in the 30 ng samples. Further, duplex read families accounted for at least 55% of the family types in the 10 ng cfDNA samples.

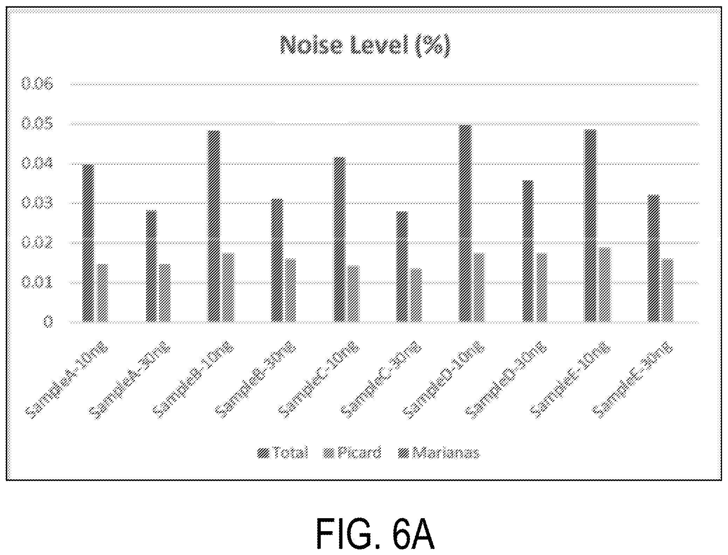

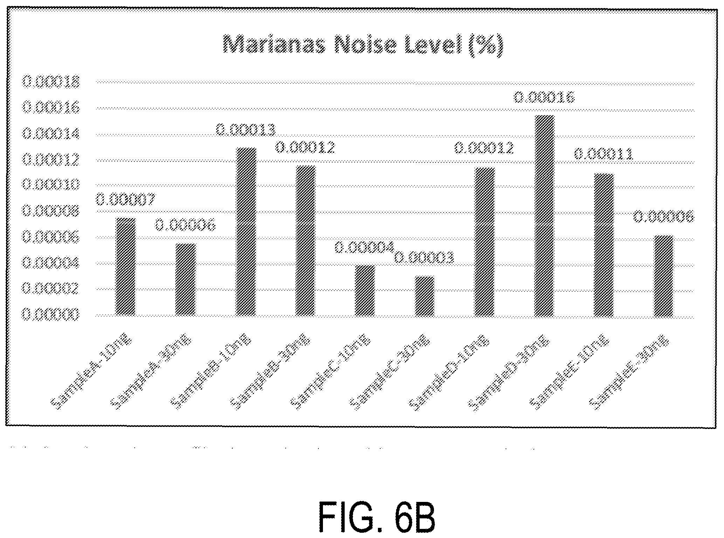

FIG. 6A shows an example of the % noise level observed before and after processing of cfDNA sequence reads (derived from different subject samples) with the Picard software (Broad Institute, Cambridge MA), where the data labeled "marianas" corresponds to the data associated with the processes and methods discussed herein. FIG. 6B shows an example of the % noise level observed when cfDNA sequence data derived from subject samples are processed using the data processing methods of the present disclosure. As shown in FIGs. 6A and 6B, the % noise level was significantly lower when the cfDNA sequence reads are processed using the data processing methods of the present disclosure.

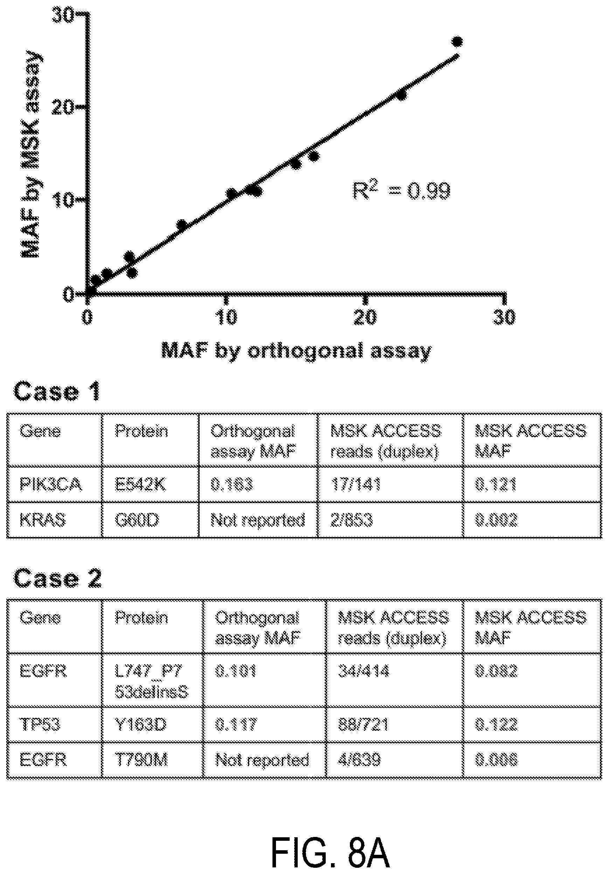

FIG. 8A shows the positive correlation between the mutant allele fractions (MAF) observed using the data processing methods disclosed herein and the MAF observed using a different (orthogonal) screening method for the same cfDNA collection. As shown in FIG. 8A, the data processing methods of the present technology identified all mutations that were reported in the orthogonal screening method (*e.g.*, PIK3CA E542K, EGFR L747_P753delinsS, and TP53 Y163D). Further, according to FIG. 8A, the data processing methods of the present technology identified additional low frequency mutations that were not reported in orthogonal screening method (e.g., KRAS G60D and EGFR T790M).

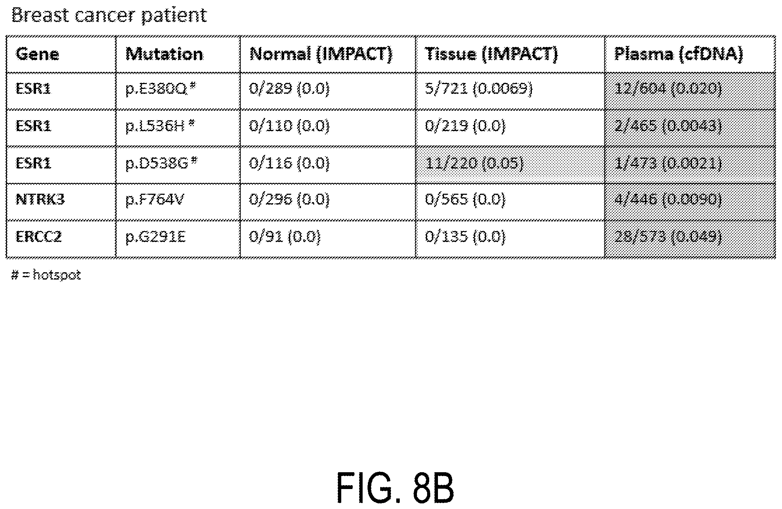

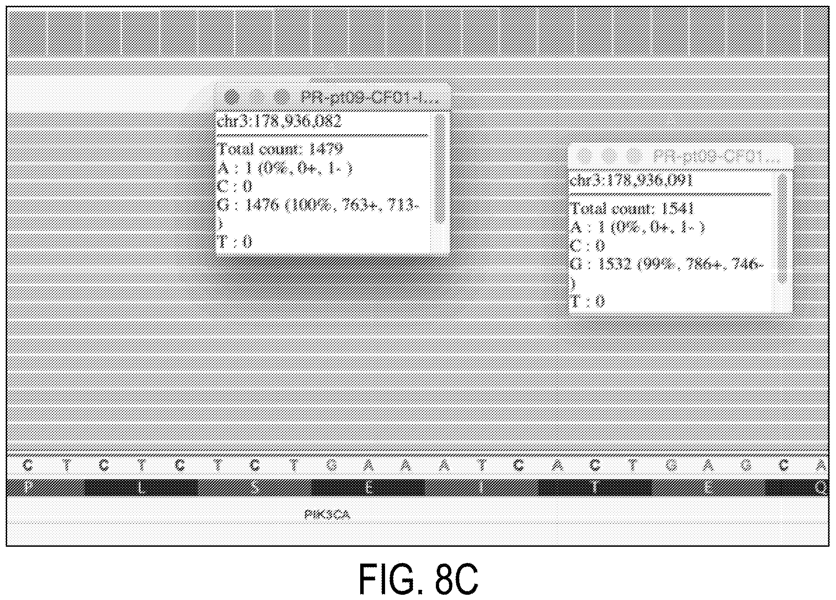

FIG. 8B illustrates an example of the variant calling results achieved with the cfDNA data processing methods disclosed herein compared to the MSK IMPACT NGS method. The MSK IMPACT data was derived from tissue biopsies that were harvested from cancer subjects. As shown in FIG. 8B, the data processing methods of the present technology identified all mutations that were reported in the MSK IMPACT method (*e.g.*, ESR1 E380Q, and ESR1 D538G). Further, according to FIG. 8A, the data processing methods of the present technology identified additional low frequency mutations that were not reported in the MSK IMPACT method (e.g., ESR1 L536H, NTRK3 F764V, and ERCC2 G291E). FIG. 8C illustrates that the cfDNA data processing methods disclosed herein correctly identified that PIK3CA E542K and E545K mutations occur in two separate DNA molecules. The presence of the mutations was confirmed using droplet digital PCR.

The methods of the present disclosure are useful for early detection of cancer, monitoring disease progression and tumor burden, identifying clinically relevant alterations and mutational signatures, detecting minimal residual disease, as well as assessing subject responsiveness or acquired resistance to a particular therapy. In one aspect, the present disclosure provides a method for monitoring cancer progression in a subject comprising: detecting the presence of at least one mutation in a cancer-related gene in a cell-free DNA (cfDNA) sample obtained from the subject using any of the computer-implemented methods described herein. Cancer progression includes metastases to secondary organs, increases in tumor volume or tumor burden, or increased tumor proliferation. The methods of the present disclosure are useful for early detection of cancer. For example, in some embodiments, the subject lacks detectable tumors.

In another aspect, the present disclosure provides a method for determining the efficacy of a therapy in a subject suffering from cancer comprising: (a) administering the therapy to the subject; (b) detecting the presence of at least one mutation in a cancer-related gene in a first cell-free DNA (cfDNA) sample obtained from the subject using any of the computer-implemented methods described herein following administration of the therapy; and (c) determining that the therapy is effective when the first cfDNA sample shows a decrease in variant allele fraction compared to that observed in a control sample obtained from the subject prior to administration of the therapy. The control sample may be a cfDNA sample or a tumor sample. The therapy may include one or more of radiation therapy, chemotherapy, surgery, immunotherapy, or surgery. Examples of chemotherapeutic agents include, but are not limited to, abraxane, capecitabine, erlotinib, fluorouracil (5-FU), gemcitabine, irinotecan, leucovorin, nab-paclitaxel, cisplatin, irinotecan, docetaxel, oxaliplatin, tipifarnib, everolimus, sunitinib, dovitinib, ruxolitinib, pegylated-hyaluronidase, pemetrexed, folinic acid, paclitaxel, MK2206, GDC-0449, IPI-926, gamma secretase/RO4929097, M402, and LY293111. Examples of immunotherapeutic agents include, but are not limited to, immune checkpoint inhibitors (e.g., antibodies targeting CTLA-4, PD-1, PD-L1), ipilimumab, 90Y-Clivatuzumab tetraxetan, pembrolizumab, nivolumab, trastuzumab, cixutumumab, ganitumab, demcizumab, cetuximab, nimotuzumab, dalotuzumab, sipuleucel-T, CRS-207, and GVAX.

### C. Computer complemented method for detecting microsatellite instability in cell-free DNA

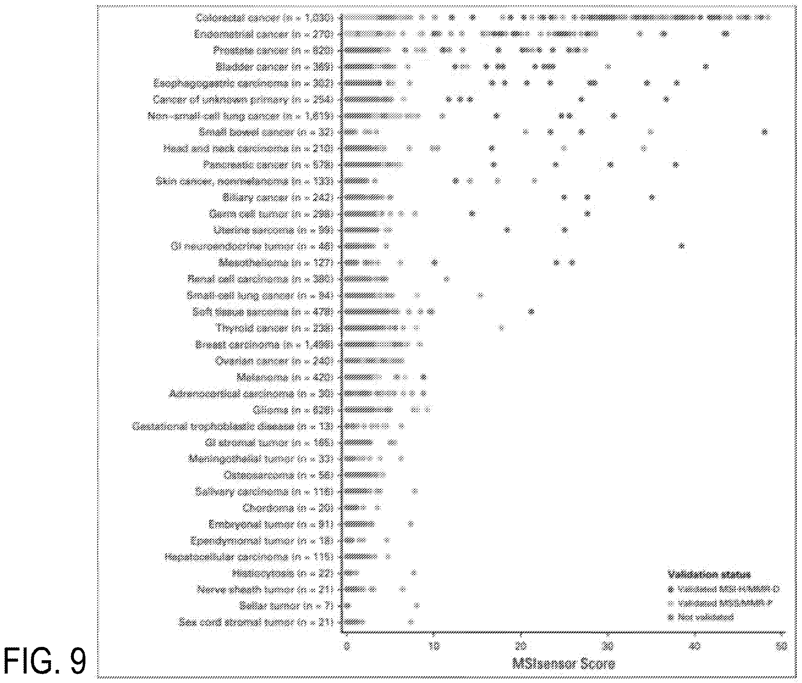

Microsatellites are short, repeated, sequences of DNA. Cancer cells that have defects in the DNA mismatch repair pathway end up accumulating errors at microsatellite regions when DNA is copied in the cell. Microsatellite instability (MSI) is a somatic genomic condition associated with impaired DNA mismatch repair (MMR) that leads to elevated mutation rates. MSI can arise sporadically in tumors due to somatic mutations in MMR-associated genes, or can arise due to the genetic condition known as Lynch Syndrome in which germline mutations in MMR-associated genes are inherited. MSI is observed in ~2-5% of solid tumors. FIG. 9 shows the landscape of MSI observed in different cancers and that MSI is frequently associated with colorectal cancer, gastrointestinal cancer, endometrial cancer, prostate cancer, and bladder cancer. In the experimental cohorts described herein, approximately 16% of the observed MSI tumors were the result of germline Lynch Syndrome mutations (Latham et al., Journal of Clinical Oncology, 2019).

The MSI signature (sporadic or inherited) is of particular clinical significance because it predicts responsiveness to immunotherapy. The immune checkpoint inhibitor pembrolizumab was approved by the FDA for all metastatic solid tumors with MSI or mismatch repair deficiency. Given the clinical significance and therapeutic relevance of MSI, it is critical that genomic profiling assays incorporate measurements of MSI. Moreover, there is evidence that MSI can be acquired later in cancer progression, so it is important to continue to monitor MSI over time.

MSI testing has traditionally been performed by PCR of 5-7 distinct 'microsatellite' sites throughout the genome. A similar condition 'mismatch repair deficiency' (MMR-d) is detected by immunohistochemistry for the proteins MLH1, MSH2, MSH6, and PMS2. Over the last few years, it has been established that MSI can be read out from next-generation sequencing of tumors using assays such as whole exome sequencing and MSK-IMPACT, a hybridization capture-based next-generation sequencing assay for targeted deep sequencing of all exons and selected introns of 341 key cancer genes in formalin-fixed, paraffin-embedded tumors (Cheng et al., J Mol Diagn. 17(3): 251-264 (2015)). Plasma cell-free DNA represents a non-invasive approach to longitudinally profile tumors. As most tumors that arise in subjects with Lynch Syndrome exhibit MSI, identification of MSI in nucleic acid (e.g., cfDNA) provides an opportunity for early detection of cancer in this high-risk population. However, while tumor sequencing is increasingly performed for MSI detection, the current methods typically fail when the tumor purity falls below ~25%.

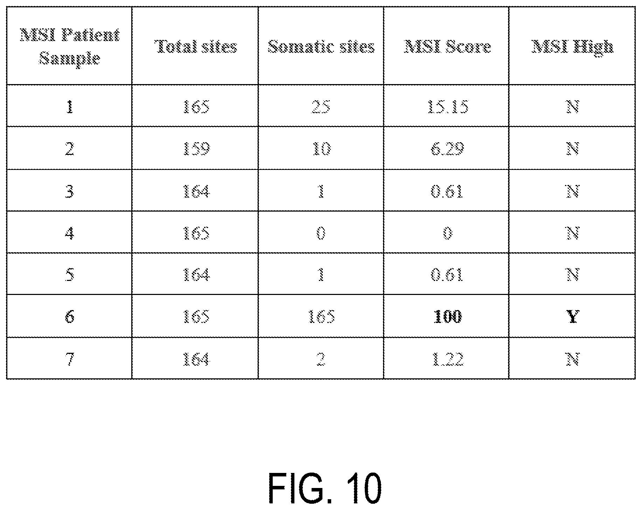

Standard NGS-based methods are expected to perform sub-optimally with respect to detecting MSI in nucleic acid (e.g., cfDNA) since the fraction of tumor-derived cfDNA in plasma is often 1% or lower, especially in early stage cancer. For example, MSIsensor is a C++ program that detects somatic microsatellite changes by computing length distributions of microsatellites per site (*i.e.,* measures variable length insertions and deletions at microsatellite regions) in paired tumor and normal sequence data, and using these length distributions to statistically compare observed distributions in both samples. See Niu et al., Bioinformatics 30(7): 1015-1016 (2014). MSIsensor was used to detect MSI signatures in tumors that were sequenced by the NGS-based MSK-IMPACT panel, which screens >1,000 microsatellite regions in the human genome. As shown in FIG. 10, only 1 out of the 7 plasma cfDNA samples obtained from MSI-High subjects (as previously determined by MSK-IMPACT assay on tumor tissue) and sequenced using MSK-IMPACT were confirmed as being MSI-High using MSIsensor. Thus, the false-negative rate of MSIsensor with respect to detecting the presence of MSI in cfDNA samples sequenced using MSK-IMPACT was 86%, which may be attributable in part to the degradation of plasma cfDNA for low-purity tumors and/or differences in read depths for tumor-normal pairs (as is often the case with cfDNA).

The data processing methods of the present disclosure are useful for detecting MSI during the early detection of cancer in subjects. Prior to detecting MSI, plasma cfDNA samples and matched white blood cell normal DNA samples are sequenced, and the corresponding sequence reads are processed using the methods described in Section B.

In some embodiments, the nucleic acid (e.g., cfDNA) sequence reads are derived from samples obtained from subjects that have an elevated risk for developing cancer, for example Lynch Syndrome subject samples. The nucleic acid (e.g., cfDNA) sequence reads derived from Lynch Syndrome subject samples may include protein-coding exons of mismatch repair genes (MSH2, MSH6, MLH1, PMS2), SNPs near the mismatch repair genes (useful in detecting allele-specific copy number (zygosity) changes), and/or at least 5, at least 10, at least 15, at least 20, at least 25, at least 30, at least 35, at least 40, at least 45, at least 50, at least 55, at least 60, at least 65, at least 70, at least 75, at least 80, at least 85, at least 90, at least 100, at least 110, at least 120, at least 130, at least 140, at least 150, at least 160, at least 170, at least 180, at least 190, at least 200, at least 300, at least 400, at least 500, at least 600, at least 700, at least 800, at least 900, or at least 1000 microsatellite regions within the human genome. See *e.g.,* Arzimanoglou et al., Cancer 82(10):1808-20 (1998); Dahiya et al., Int J Cancer. 72(5):762-7 (1997). In certain embodiments, the subject suffers from, or is suspected of having Lynch Syndrome, and/or harbors at least one mutation in one or more mismatch repair genes selected from the group consisting of MSH2, MSH6, MLH1, and PMS2. Additionally, or alternatively, in some embodiments, the subject suffers from or is at risk for ovarian cancer, breast cancer, colorectal cancer, lung cancer, prostate cancer, gastric cancer, pancreatic cancer, cervical cancer, liver cancer, bladder cancer, cancer of the urinary tract, thyroid cancer, renal cancer, carcinoma, melanoma, head and neck cancer, or brain cancer.

Additionally, or alternatively, in some embodiments, the method further comprises determining the presence of at least one mutation in an exon of a cancer-related gene selected from the group consisting of:
AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3, GNA11, GNAQ, GNAS, H3F3A, HIST1H3B, HRAS, IDH1, IDH2, IKZF1, INPPL1, JAK 1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL, and XPO1.
The at least one mutation may be a deletion, an insertion, a translocation, an inversion, a copy number variant, or a point mutation. Additionally, or alternatively, in some embodiments, the method further comprises determining the presence of at least one genomic alteration in an intron of a cancer-related gene selected from the group consisting of: ALK, BRAF, EGFR. ETV6, FGFR2, FGFR3, MET, NTRK1, RET and ROS1 or a promoter region of TERT. The cfDNA sample may be serum, plasma, sweat, tears, urine, saliva, synovial fluid, lymphatic fluid, ascites fluid, amniotic fluid, or interstitial fluid.

In another aspect, the present disclosure provides a method for monitoring cancer progression in a subject comprising: detecting the presence of microsatellite instability in nucleic acid (e.g., cfDNA) sample obtained from the subject using any of the computer-implemented methods described herein. Cancer progression includes metastases to secondary organs, increases in tumor volume or tumor burden, or increased tumor proliferation. The methods of the present disclosure are useful for early detection of cancer. For example, in some embodiments, the cfDNA sample does not comprise a mutation or genomic alteration in any cancer-related gene described herein. Additionally or alternatively, in some embodiments, the subject lacks detectable tumors.

In one aspect, the present disclosure provides a method for determining the efficacy of a therapy in a subject with a MSI-High tumor comprising: (a) administering the therapy to the subject; (b) detecting the presence of microsatellite instability in a first nucleic acid (e.g., cfDNA) sample obtained from the subject using any of the computer-implemented methods described herein following administration of the therapy; and (c) determining that the therapy is effective when the first nucleic acid (e.g., cfDNA) sample shows a shift towards a distance metric that is associated with microsatellite stability (MSS) compared to that observed in a control sample obtained from the subject prior to administration of the therapy. The control sample may be a nucleic acid (e.g., cfDNA) sample or a tumor sample. The therapy may include one or more of radiation therapy, chemotherapy, surgery, immunotherapy, or surgery. Examples of chemotherapeutic agents include, but are not limited to, abraxane, capecitabine, erlotinib, fluorouracil (5-FU), gemcitabine, irinotecan, leucovorin, nab-paclitaxel, cisplatin, irinotecan, docetaxel, oxaliplatin, tipifarnib, everolimus, sunitinib, dovitinib, ruxolitinib, pegylated-hyaluronidase, pemetrexed, folinic acid, paclitaxel, MK2206, GDC-0449, IPI-926, gamma secretase/RO4929097, M402, and LY293111. Examples of immunotherapeutic agents include, but are not limited to, immune checkpoint inhibitors (e.g., antibodies targeting CTLA-4, PD-1, PD-L1), ipilimumab, 90Y-Clivatuzumab tetraxetan, pembrolizumab, nivolumab, trastuzumab, cixutumumab, ganitumab, demcizumab, cetuximab, nimotuzumab, dalotuzumab, sipuleucel-T, CRS-207, and GVAX.

### Examples

Microsatellite regions are some of the most error-prone sites in the genome. These Examples demonstrate that the ultra-high depth sequencing and UMI-based error-suppression achieved using the methods described in Section B and Section C significantly improved the sensitivity for detecting MSI.

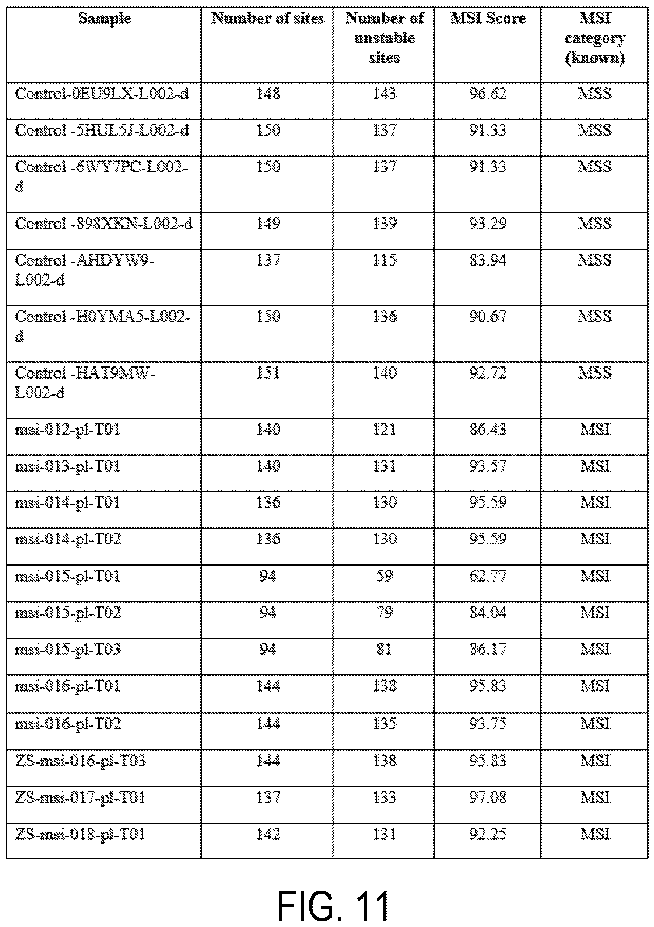

Based on a reanalysis of >20,000 tumors sequenced by the MSK-IMPACT assay, a small subset of 165 (out of >1,000) of the most frequently mutated microsatellite regions were selected. MSI Score is based on an analysis that looks for DNA slippage (variable length insertions and deletions) at microsatellite regions. The score reflects the % of microsatellite regions with significantly more insertions/deletions in a tumor sample compared to a matched normal sample. The existing form of MSIsensor was used to detect the presence of MSI in nucleic acid (e.g., cfDNA) samples. As shown in FIG. 11, MSIsensor in its current form failed to adequately discriminate between MSI-High and MSS (microsatellite stable) cases when analyzing nucleic acid (e.g., cfDNA) data.

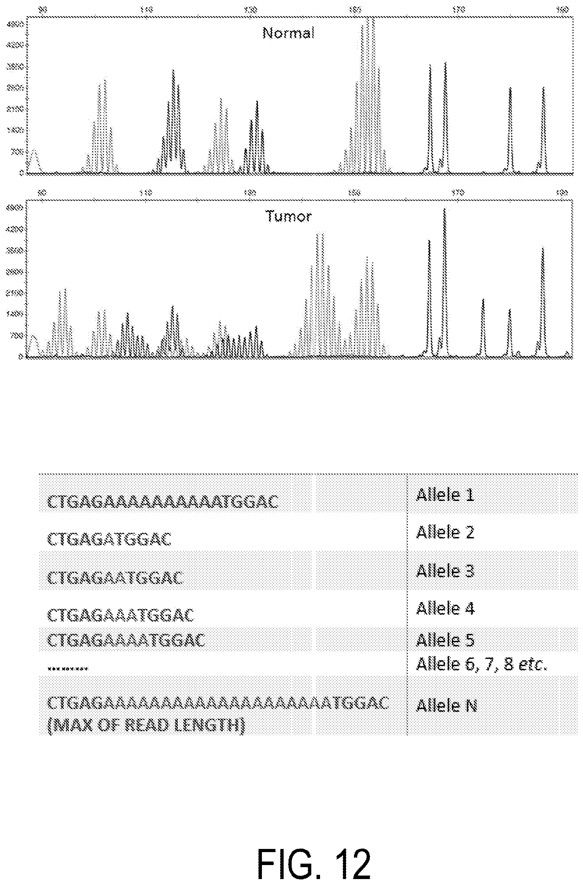

Plasma cfDNA samples and matched white blood cell normal DNA samples were deep-sequenced, and the corresponding sequence reads were processed using the methods described in Section B. The MSI detection algorithm disclosed herein directly compares the number of individual sequence reads observed for every possible allele (1 to N) at each of the 165 microsatellite sites. A vector of length N (upper limit was set as the largest possible read length) was created for each microsatellite site, and a distance metric was computed between plasma cfDNA and matched WBC samples after a per-sample, per-locus normalization was carried out. See FIG. 12. The 165 distance metrics were aggregated to form a distribution for the plasma cfDNA-matched WBC pair. In an exemplary approach, a second distribution can be generated for the same microsatellite loci but from cfDNA of a different sample without MSI. The two distributions can be compared to determine or detect the presence of MSI in the subjects cfDNA. In some examples, machine learning tools can be utilized to detect MSI in a sample. As an example, trained classifiers can be used to determine whether the first distribution indicates the presence of MSI. The classifiers may determine the presence of MSI in the first distribution independently of the second distribution. A classifier such as, for example, a support vector machine (SVM) was used to distinguish MSI from MSS cases.

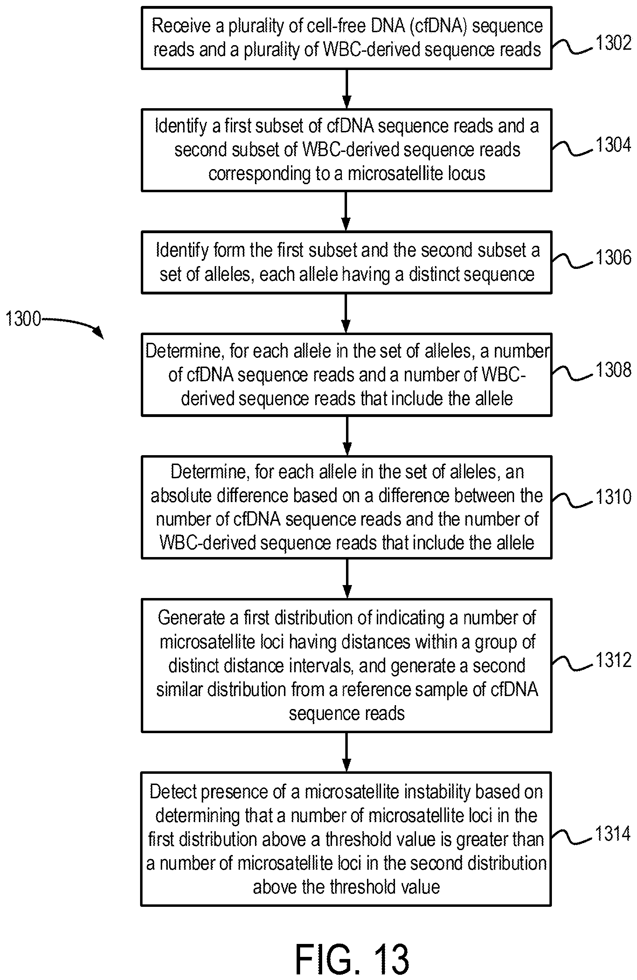

FIG. 13 shows a flow diagram of an example process 1300 for determining the presence of microsatellite instability in nucleic acid (e.g., cfDNA) samples. In particular, the process 1300 can be utilized to analyze cfDNA sequence reads of a subject, and update a database to associate an identifier of the subject with the presence of microsatellite instability. The process 1300 can be executed by the genomic data processing system 120 shown in FIG. 1C. The genomic data processing system 120 can include or execute on one or more processors and can include scripts, modules, or computer-executable code, which when executed by one or more processors, can cause the genomic data processing system 120 to perform the process 1300. The process 1300 includes receiving, by one or more processors, from a next generation sequencing device, a plurality of cfDNA sequence reads and a plurality of WBC-derived sequence reads that are derived from a subject (1302). The cfDNA sequence reads and the WBC derived sequence reads can each include a forward unique molecular identifier (UMI) and a reverse UMI, where the forward and the reverse UMIs can be serve as an identifier for the subject. In some instances, the cfDNA sequence reads and the WBC-derived sequence reads can include both top and bottom strand sequence reads.

The process 1300 can select a microsatellite locus from a plurality of microsatellite loci for further processing of the sequence reads. For example, the process 1300 can include, for each microsatellite loci, identifying a first subset of cfDNA sequence reads and a second subset of WBC-derived sequence reads corresponding to a microsatellite locus. Thus, both the first subset and the second subset include sequence reads that correspond to the same microsatellite loci.

The process 1300 includes identifying from the first subset and the second subset, a set of alleles, each allele of the set of alleles having a distinct sequence (1306). One example set of alleles is shown in FIG. 12, which shows alleles includes Allele 1 to Allele N. The one or more processors can compare the cfDNA sequence reads in the first subset with a number of alleles, and compare the WBC-derived sequence reads in the second subset also with a number of alleles. The set of alleles can be alleles that are identified as being present in the sequence reads in both the first subset and the second subset.

The process 1300 includes determining, for each allele of the set of alleles, a number of cfDNA sequence reads and a number of WBC-derived sequence reads that include the allele (1308). For example, for Allele 1, the one or more processors, can determine the number of cfDNA sequence reads in the first subset that include Allele 1. Similarly, for Allele 1, the one or more processors can determine the number of WBC-derived sequence reads that include Allele 1. In a similar manner, the one or more processor can determine the number of sequence reads in each of the first and second subsets that include each allele in the set of alleles. Generally, the one or more processors can determine a number hₜᵢ denoting a number of cfDNA sequence reads corresponding to an Allele i, and can determine a number hₙᵢ denoting a number of WBC-derived sequence reads corresponding to the Allele i.

In some instances, the one or more processors can normalize the number of cfDNA sequence reads and the number of WBC-derived sequence reads. For example, the one or more processors can determine a normalized value hₙₜᵢ by dividing the value hₜᵢ by a sum of the number of cfDNA sequence reads for all alleles (Σ*ᵢ hₜᵢ*). Similarly, the one or more processors can determine a normalized value hₙₙᵢ by dividing the value hₙᵢ by the sum of the number of WBC-derived sequence reads for all alleles (Σ*ᵢ hₙᵢ*)*.*

The process 1300 further includes determining, by the one or more processors, an absolute difference based on a difference between the number of cfDNA sequence reads for the allele and the number of WBC-derived sequence reads for the allele (1310). In particular, the one or more processors can, for each allele i, determine an absolute difference aᵢ between the corresponding number (hₜᵢ) of cfDNA sequence reads for that allele and the number (hₙᵢ) of WBC-derived sequence reads for that allele. Thus, the absolute difference aᵢ can be determined based on: |hₜᵢ - hₙᵢ|. In some instances, the absolute difference aᵢ can be determined based on the normalized values. For example, the absolute difference aᵢ can be determined based on: |hₙₜᵢ - hₙₙᵢ|.

The process 1300 includes determining, for each microsatellite locus, from the plurality of microsatellite loci, a distance based on a sum of absolute differences associated with all alleles in the set of alleles (1310). As mentioned above, the set of alleles are associated with a microsatellite locus. To determine the distance, the one or more processors can add the absolute differences aᵢ associated with all alleles. In particular, the one or more processors can determine a distance d for a microsatellite loci based on Σ*ᵢ* a*ᵢ*. Assuming that there are m number of microsatellite loci, the one or more processors can determine m distance values d for a microsatellite locus. For example, the one or more processors can determine distances d₁, d₂, d₃, ... , dₘ corresponding to the m number of microsatellite loci.

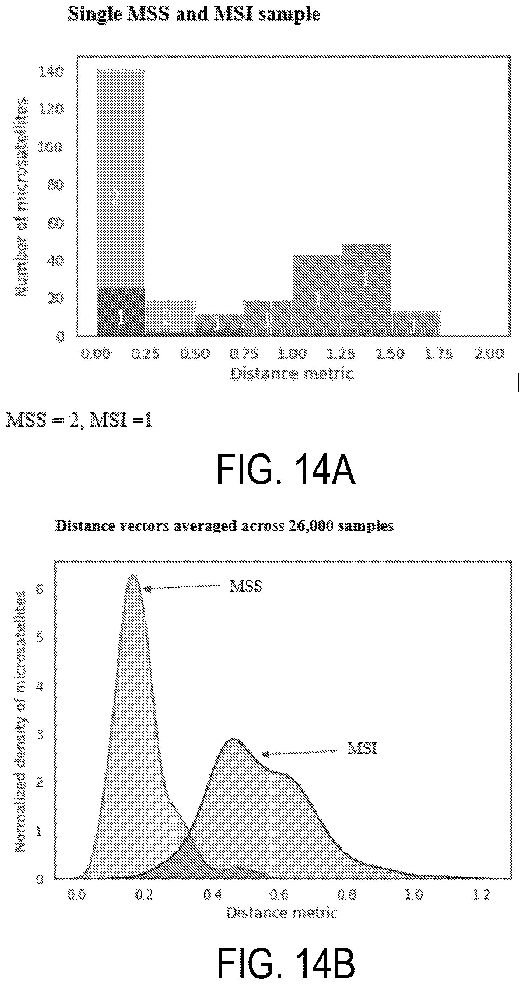

The process 1300 also includes generating, by the one or more processors, a first distribution indicating a number of microsatellite loci having distances within a group of distinct distance intervals (1312). The one or more processors can generate a frequency distribution of the distance values over a group of distance intervals. Example distributions are shown in FIG. 14A and 14B. In particular, FIG. 14A shows a first distribution (indicated by the label "1") associated with the frequency distribution of the distance values determined for the various microsatellite loci over a group of distinct distance intervals 0-0.25, 0.25-0.5, 0.5-1.0, and so on. As an example, the first frequency distribution shows about 40 microsatellite loci having distance values between the range 1.0 and 1.25. FIG. 14B shows another example distribution (labeled "MSI") showing a normalized density distributions of microsatellites over various distance values of a large number of MSI tumors.

The process 1300 includes generating, by the one or more processors, a second distribution indicating a number of microsatellite loci having distances within the group of distinct distance intervals, where the second distribution is derived from distances associated with each microsatellite locus observed in a reference sample (1312). In particular, the reference samples can include cfDNA sequence reads and WBC-derived sequence reads from a reference subject. The process discussed above for determining the distance values for the microsatellite loci in samples associated with the subject can be similarly applied to the samples from the reference subject to determine the second distribution. Example second distributions associated with the reference samples are shown in FIGS. 14A and 14B. In particular, the second distribution is labeled "2" in FIG. 14A and labeled "MSS" in FIG. 14B.

The process 1300 includes determining, by the one or more processors, that a number of microsatellite loci in the first distribution above a threshold value is greater than a number of microsatellite loci in the second distribution above the threshold value to detect the presence of microsatellite instability (1314). For example, referring to FIG. 14B, an example threshold value of 0.4 can be selected, and the number of microsatellite loci above 0.4 in the first distribution can be compared with the number of microsatellite loci above 0.4 in the second distribution. If the number in the first distribution is greater than the number in the second distribution, the one or more processors can detect the presence of microsatellite instability.

In some instances, the one or more processors can adopt other methods to detect the presence of microsatellite instability from the first and the second distribution. In one example, the one or more processors use a Z-test statistic to compare the first distribution to the second distribution, and detect the presence of microsatellite instability if the score of the Z-test is above a threshold value. A larger score can indicate that the first distribution, which associated with the subject, is different from the second distribution, which is associated with a reference subject.

In some examples, the one or more processors can adopt machine learning techniques to detect the presence of microsatellite instability. For example, the one or more processors can utilize a classifier, such as, for example, a support vector machine (SVM), to determine whether the first distribution can be classified as having microsatellite instability. The classifier can be trained with data that is labeled with either the presence of lack of microsatellite instability. The classifier can build a model based on that data. Based on the model, the classifier can determine whether the first distribution can be classified as having the presence of microsatellite instability or no presence of microsatellite instability. The SVM is a non-probabilistic binary (linear or non-linear) classifier where examples are mapped onto a space such that examples of separate categories are divided by a clear gap that is as wide as possible. A new example, such as the first distribution, can be mapped onto the same space and predicted as belonging to the presence or no presence of microsatellite instability. The one or more processors feed data to an SVM to enable classification. The data can include, for example, distributions that indicate the presence of microsatellite instability and distributions that indicate no presence of microsatellite instability. The SVM can construct a hyperplane in a multi-dimensional space, which can be used for classification or regression. In some examples, the one or more processors can utilize other types of classifiers such as, for example, linear classifiers, quadratic classifiers, kernel estimators, neural networks, learning vector quantization, etc., to classify the first distribution as having microsatellite instability or not having microsatellite instability.

The process 1300 can further include sorting in one or more data structure, an association between the subject and the presence of microsatellite instability. For example, the one or more processors can store data structure similar to that shown in FIG. 10 in memory. Responsive to determining the presence of microsatellite instability, the one or more processors can update the data structure to include an indicator such as "Y" under the MSI high column to store the association of the presence of MSI and the identity of the subject.

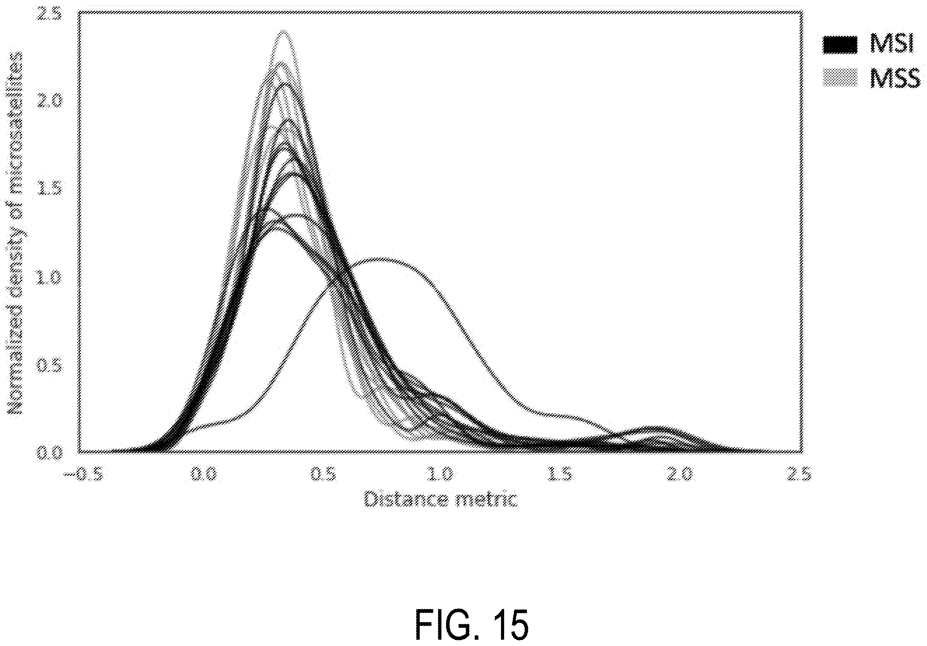

*Results.* The MSI detection model (Allelic Distance-based Microsatellite Instability Estimator or ADMIE) was trained using MSK-IMPACT results from 311 tumor tissue samples with confirmatory immunohistochemistry or PCR to establish the MSI status. Computed allelic distances were used to predict MSI/MSS status for a 'held-out' test set of MSK-IMPACT data from over 26,000 tumor tissues (FIGs. 14A-14B), and for an independent test set of data from plasma cfDNA samples (FIGs. 15-16). As shown in FIGs. 14A-14B, MSI tumor samples exhibited larger allelic distances relative to MSS samples. FIG. 15 shows the distance metric distributions for 7 plasma cfDNA samples from subjects with MSS tumors (gray) and 12 plasma cfDNA samples from subjects with MSI tumors (black). While the distributions are similar due to the low tumor fractions of the cfDNA samples, the MSI cfDNA samples generally show a rightward shift towards greater allelic distances, thereby permitting the SVM classifier to accurately and reliably discriminate between MSI and MSS cfDNA samples. The distance from the SVM decision boundary is shown on FIG. 16. For every case, tumors were also sequenced using the MSK-IMPACT assay, and at least one tumor mutation was present within the target regions captured by NGS-screening of the cfDNA samples. These mutations were used to determine the fraction of tumor cfDNA within the plasma, as estimated by the mean variant allele fraction (VAF) observed at the corresponding genomic sites. The majority of MSI-positive cases exhibited VAFs suggestive of very low tumor content (<1%), with some cases harboring no evidence of the tumor mutation(s), demonstrating that MSI detection was even more sensitive than mutation detection.

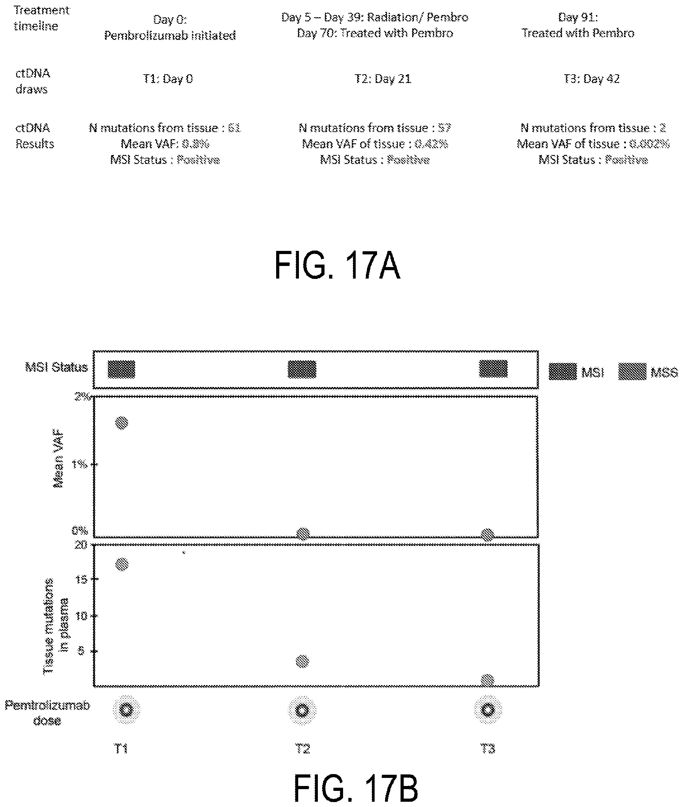

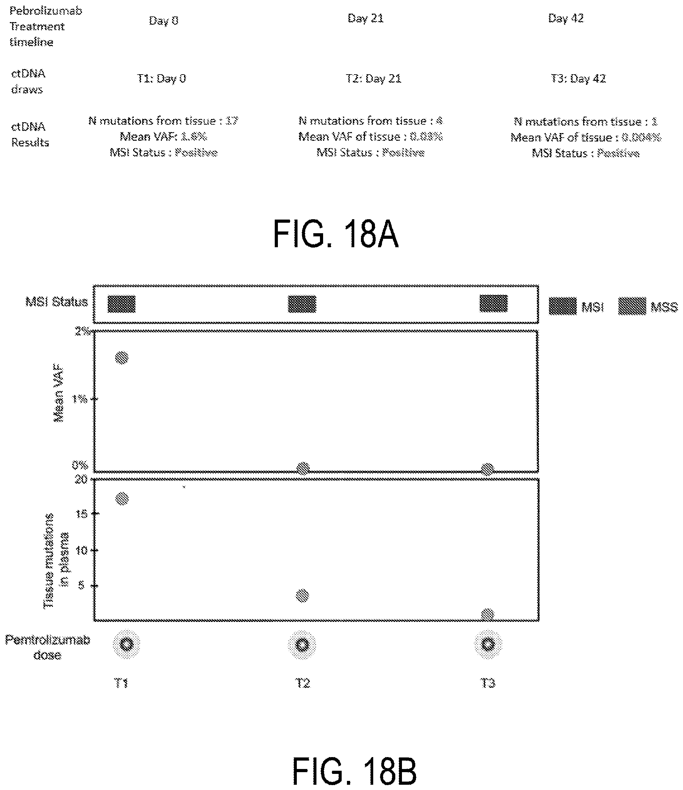

FIGs. 17A-17B and 18A-18B show examples of two subjects with Lynch syndrome and MSI-High tumors (stage III-C rectal cancer). Three plasma samples were collected from both subjects at separate time points relative to the administration of immunotherapy or chemo-radiation. For each subject, the number of detectable mutations and the VAF of the mutations successively decreased as the subjects responded to treatment. ADMIE was able to detect MSI even in post-treatment samples.

These results demonstrate that the data processing methods and systems disclosed herein are useful for detecting cancer-related mutations and microsatellite instability in cell-free DNA (cfDNA) sequence data with a high degree of accuracy and sensitivity.

The term "adapter" refers to a short, chemically synthesized, nucleic acid sequence which can be used to ligate to the end of a nucleic acid sequence in order to facilitate attachment to another molecule. The adapter can be single-stranded or double-stranded. An adapter can incorporate a short (typically less than 50 base pairs) sequence useful for PCR amplification or sequencing. In some embodiments, the adapter includes a unique molecular identifier.

The term "hold out" in the context of machine learning refers to splitting up a dataset into a 'training set' and 'test set'. The training set is used to train a model, and the test set is used to see how well that model performs on unseen data.

The terms "variant allele fraction," "VAF," "mutant allele fraction" or "MAF" refer to fractions of a mutant allele over the total number of mutant (alternate allele) plus wild-type alleles (reference allele).

"Unique molecular identifiers" or "UMIs" are random nucleotide sequences used to tag each DNA molecule (fragment) prior to library amplification, thereby aiding in the identification of PCR duplicates. If two reads align to the same location and have the same UMI, it is highly likely that they are PCR duplicates originating from the same DNA molecule prior to amplification. As a result, all sequence reads with identical genomic coordinates and UMIs can be collapsed into a single representative read, which is useful for obtaining an accurate estimate of the relative concentration of the DNA molecules in the DNA sample.

The term "plurality of first DNA reads" refers to DNA sequence reads that are derived from the first oligonucleotide strand (e.g., sense strand) of a double-stranded DNA molecule. In some embodiments, the plurality of first DNA reads originate from cfDNA or white blood cells (WBC).

The term "plurality of second DNA reads" refers to DNA sequence reads that are derived from the second oligonucleotide strand (*e.g.*, anti-sense strand) of a double-stranded DNA molecule. The plurality of second DNA reads may be at least partially or completely complementary to the plurality of first DNA reads (*e.g.,* at least 70%. 75%, 80%, 85%, 90%, or 95% complementary). In some embodiments, the plurality of second DNA reads originate from cfDNA or white blood cells (WBC). The term "white blood cells" or "WBC" refers to blood cells that are colorless, lack hemoglobin, contain a nucleus, and include lymphocytes, monocytes, neutrophils, eosinophils, and basophils.

The terms "complementary" or "complementarity" as used herein with reference to polynucleotides (i.e., a sequence of nucleotides such as an oligonucleotide or a target nucleic acid) refer to the base-pairing rules. The complement of a nucleic acid sequence as used herein refers to an oligonucleotide which, when aligned with the nucleic acid sequence such that the 5' end of one sequence is paired with the 3' end of the other, is in "antiparallel association." For example, the sequence "5'-A-G-T-3'" is complementary to the sequence "3'-T-C-A-5." Complementarity need not be perfect; stable duplexes may contain mismatched base pairs, degenerative, or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs.

"Coverage" or "depth" as used herein refers to the number of reads that align to, or "cover," known reference bases. The next-generation sequencing (NGS) coverage level often determines whether variant discovery can be made with a certain degree of confidence at particular base positions.

"Next-generation sequencing or NGS" as used herein, refers to any sequencing method that determines the nucleotide sequence of either individual nucleic acid molecules (e.g., in single molecule sequencing) or clonally expanded proxies for individual nucleic acid molecules in a high throughput parallel fashion (e.g., greater than 103, 104, 105 or more molecules are sequenced simultaneously). In one embodiment, the relative abundance of the nucleic acid species in the library can be estimated by counting the relative number of occurrences of their cognate sequences in the data generated by the sequencing experiment. Next generation sequencing methods are known in the art. Examples of Next Generation Sequencing techniques include, but are not limited to pyrosequencing, Reversible dye-terminator sequencing, SOLiD sequencing, Ion semiconductor sequencing, Sequencing by synthesis (SBS), Helioscope single molecule sequencing etc. Next generation sequencing methods can be performed using commercially available kits and instruments from companies such as the Life Technologies/Ion Torrent PGM or Proton, the Illumina HiSEQ or MiSEQ, and the Roche/454 next generation sequencing system.

As used herein, "oligonucleotide" refers to a molecule that has a sequence of nucleic acid bases on a backbone comprised mainly of identical monomer units at defined intervals. The bases are arranged on the backbone in such a way that they can bind with a nucleic acid having a sequence of bases that are complementary to the bases of the oligonucleotide. The most common oligonucleotides have a backbone of sugar phosphate units. A distinction may be made between oligodeoxyribonucleotides that do not have a hydroxyl group at the 2' position and oligoribonucleotides that have a hydroxyl group at the 2' position. Oligonucleotides of the method which function as primers or probes are generally at least about 10-15 nucleotides long and more preferably at least about 15 to 35 nucleotides long, although shorter or longer oligonucleotides may be used in the method. The exact size will depend on many factors, which in turn depend on the ultimate function or use of the oligonucleotide.

As used herein, a "sample" refers to a substance that is being assayed for the presence of a mutation in cfDNA, *e.g.*, ctDNA. Processing methods to release or otherwise make available a nucleic acid for detection are well known in the art and may include steps of nucleic acid manipulation. A sample may be a body fluid. In some cases, a biological sample may consist of or comprise serum, plasma, sweat, tears, urine, saliva, synovial fluid, lymphatic fluid, ascites fluid, amniotic fluid, or interstitial fluid, cerebral spinal fluid, and the like.

## Claims

1. A computer-implemented method, comprising:
receiving, by one or more processors, from a next generation sequencing device:
(i) a plurality of cell-free DNA (cfDNA) sequence read-pairs derived from a subject, each cfDNA sequence read from the plurality of cfDNA sequence reads including either a forward unique molecular identifier (UMI) or a reverse UMI, and
(ii) a plurality of WBC-derived sequence read-pairs derived from the subject, each WBC-derived sequence read from the plurality of WBC-derived sequence reads optionally including the forward UMI or the reverse UMI;
for each microsatellite locus of a plurality of microsatellite loci,
identifying, by the one or more processors, a first subset of the plurality of cfDNA sequence reads and a second subset of the plurality of WBC-derived sequence reads, each read in the first subset and the second subset corresponds to the microsatellite locus;
identifying, by the one or more processors, from the first subset and the second subset, a set of alleles, each allele of the set of alleles having a distinct sequence;
determining, by the one or more processors, for each allele of the set of alleles, a number of cfDNA sequence reads that include the allele;
determining, by the one or more processors, for each allele of the set of alleles, a number of WBC-derived sequence reads that include the allele;
determining, by the one or more processors, for each allele in the set of alleles, an absolute difference based on a difference between the number of cfDNA sequence reads for the allele and the number of WBC-derived sequence reads for the allele,
determining, by the one or more processors, for each microsatellite locus from the plurality of microsatellite loci, a distance based on a sum of absolute differences associated with all alleles in the set of alleles;
generating, by the one or more processors, a first distribution indicating a number of microsatellite loci having distances within a group of distinct distance intervals;
generating, by the one or more processors, a second distribution indicating a number of microsatellite loci having distances within the group of distinct distance intervals, the second distribution derived from distances associated with each microsatellite locus of the plurality of microsatellite loci observed in a reference sample;
determining, by the one or more processors, that a number of microsatellite loci in the first distribution above a threshold distance metric is greater than a number of microsatellite loci in the second distribution above the threshold distance metric to detect a presence of microsatellite instability in the subject; and
storing, by the one or more processors, responsive to the determination, in one or more data structures, an association between the subject and the presence of microsatellite instability.

2. The computer-implemented method of claim 1, further comprising:
normalizing, by the one or more processors, for each allele of the set of alleles, the number of cfDNA sequence reads that include the allele based on a sum of the number of cfDNA sequence reads corresponding to all alleles in the set of alleles to generate a respective normalized number of cfDNA sequence reads corresponding to the allele;
normalizing, by the one or more processors, for each allele of the set of alleles, the number of WBC-derived sequences that include the allele based on a sum of the number of WBC-derived sequence reads corresponding to all alleles in the set of alleles to generate a respective normalized number of WBC-derived sequence reads corresponding to the allele;
wherein, for each allele in the set of alleles, the absolute difference is based on a difference between the normalized number of cfDNA sequence reads for the allele and the normalized number of WBC-derived sequence reads for the allele, optionally wherein the sum of absolute differences associated with all alleles in the set of alleles is based on a sum of an absolute difference between normalized number of cfDNA sequence reads and normalized number of WBC-derived sequence reads for each allele in the set of alleles.

3. The computer-implemented method of any one of claims 1-2, wherein the cfDNA sample is serum, plasma, sweat, tears, urine, saliva, synovial fluid, lymphatic fluid, ascites fluid, amniotic fluid, or interstitial fluid or wherein the subject
suffers from, or is suspected of having Lynch Syndrome;
harbors at least one mutation in one or more mismatch repair genes selected from the group consisting of MSH2, MSH6, MLH1, and PMS2; and/or
suffers from or is at risk for ovarian cancer, breast cancer, colorectal cancer, lung cancer, prostate cancer, gastric cancer, pancreatic cancer, cervical cancer, liver cancer, bladder cancer, cancer of the urinary tract, thyroid cancer, renal cancer, carcinoma, melanoma, head and neck cancer, or brain cancer.

4. The computer-implemented method of any one of claims 1-2 or 4, further comprising
determining the presence of at least one mutation in an exon of a cancer-related gene selected from the group consisting of: AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3, GNA11, GNAQ, GNAS, H3F3A, HIST1H3B, HRAS, IDH1, IDH2, IKZF1, INPPL1, JAK1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL, and XPO1, optionally wherein the at least one mutation is a deletion, an insertion, a translocation, an inversion, a copy number variant, or a point mutation; and/or
determining the presence of at least one genomic alteration in an intron of a cancer-related gene selected from the group consisting of: ALK, BRAF, EGFR. ETV6, FGFR2, FGFR3, MET, NTRK1, RET and ROS1 or a promoter region of TERT.

5. The computer-implemented method of claim 1, further comprising:
generating, by the one or more processors, a machine-learning or statistical classifier that generates a decision boundary on a coordinate space that separates a first set of data points that represent presence of microsatellite instability in sequence reads and a second set of data points that represent no presence of microsatellite instability in sequence reads;
processing, by the one or more processors, the first distribution using the classifier to determine whether the first distribution belongs to the first set of data points or to the second set of data points; and
determining, by the one or more processors, microsatellite instability responsive to the classifier classifying the first distribution as belonging to the first set of data points that represent presence of microsatellite instability, optionally wherein the classifier includes a support vector machine (SVM).

6. A method for monitoring cancer progression in a subject comprising:
detecting the presence of microsatellite instability in a cell-free DNA (cfDNA) sample obtained from the subject using the computer-implemented method of any of the preceding claims, optionally wherein
cancer progression includes metastases to secondary organs, increases in tumor volume or tumor burden, or increased tumor proliferation, or
the cfDNA sample does not comprise a mutation in a cancer-related gene selected from the group consisting of: AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3, GNA11, GNAQ, GNAS, H3F3A, HIST1H3B, HRAS, IDH1, IDH2, IKZF1, INPPL1, JAK1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL, and XPO1, or
the cfDNA sample does not comprise a genomic alteration in a cancer-related gene selected from the group consisting of: ALK, BRAF, EGFR. ETV6, FGFR2, FGFR3, MET, NTRK1, RET and ROS1 or a promoter region of TERT, or
the subject lacks detectable tumors.

7. A method for determining the efficacy of a therapy in a subject with a MSI-High tumor comprising:
providing a first cell-free DNA (cfDNA) sample obtained from the subject after the therapy has been administered;
detecting the presence of microsatellite instability in the first cfDNAsample using the computer-implemented method of any one of claims 1-5; and
determining that the therapy is effective when the first cfDNA sample shows a shift towards a distance metric that is associated with microsatellite stability (MSS) compared to that observed in a control sample obtained from the subject prior to administration of the therapy.

8. The method of claim 7, wherein the therapy is one or more of radiation therapy, chemotherapy, surgery, immunotherapy, or surgery.

9. The method of claim 8, wherein chemotherapy includes the administration of one or more chemotherapeutic agents selected from the group consisting of abraxane, capecitabine, erlotinib, fluorouracil (5-FU), gemcitabine, irinotecan, leucovorin, nab-paclitaxel, cisplatin, irinotecan, docetaxel, oxaliplatin, tipifarnib, everolimus, sunitinib, dovitinib, ruxolitinib, pegylated-hyaluronidase, pemetrexed, folinic acid, paclitaxel, MK2206, GDC-0449, IPI-926, gamma secretase/RO4929097, M402, and LY293111.

10. The method of claim 8, wherein immunotherapy includes the administration of one or more agents selected from the group consisting of immune checkpoint inhibitors (e.g., antibodies targeting CTLA-4, PD-1, PD-L1), ipilimumab, 90Y-Clivatuzumab tetraxetan, pembrolizumab, nivolumab, trastuzumab, cixutumumab, ganitumab, demcizumab, cetuximab, nimotuzumab, dalotuzumab, sipuleucel-T, CRS-207, and GVAX.

11. A system, comprising:
one or more processors, configured to:
receive from a next generation sequencing device:
(i) a plurality of cell-free DNA (cfDNA) sequence read-pairs derived from a subject, each cfDNA sequence read from the plurality of cfDNA sequence reads including either a forward unique molecular identifier (UMI) or a reverse UMI, and
(ii) a plurality of WBC-derived sequence reads derived from the subject, each WBC-derived sequence read from the plurality of WBC-derived sequence reads optionally including the forward UMI or the reverse UMI;
for each microsatellite locus of a plurality of microsatellite loci,
identify a first subset of the plurality of cfDNA sequence reads and a second subset of the plurality of WBC-derived sequence reads, each read in the first subset and the second subset corresponds to the microsatellite locus;
identify from the first subset and the second subset, a set of alleles, each allele of the set of alleles having a distinct sequence;
determine, for each allele of the set of alleles, a number of cfDNA sequence reads that include the allele;
determine, for each allele of the set of alleles, a number of WBC-derived sequence reads that include the allele;
determine, for each allele in the set of alleles, an absolute difference based on a difference between the number of cfDNA sequence reads for the allele and the number of WBC-derived sequence reads for the allele,
determine, for each microsatellite locus from the plurality of microsatellite loci, a distance based on a sum of absolute differences associated with all alleles in the set of alleles;
generate a first distribution indicating a number of microsatellite loci having distances within a group of distinct distance intervals;
generate a second distribution indicating a number of microsatellite loci having distances within the group of distinct distance intervals, the second distribution derived from distances associated with each microsatellite locus of the plurality of microsatellite loci observed in a reference sample;
determine that a number of microsatellite loci in the first distribution above a threshold distance metric is greater than a number of microsatellite loci in the second distribution above the threshold distance metric to detect a presence of microsatellite instability in the subject; and
store, responsive to the determination, in one or more data structures, an association between the subject and the presence of microsatellite instability.

12. The system of claim 11, wherein the one or more processors are configured to:
normalize, for each allele of the set of alleles, the number of cfDNA sequence reads that include the allele based on a sum of the number of cfDNA sequence reads corresponding to all alleles in the set of alleles to generate a respective normalized number of cfDNA sequence reads corresponding to the allele;
normalize, for each allele of the set of alleles, the number of WBC-derived sequence that include the allele based on a sum of the number of WBC-derived sequence reads corresponding to all alleles in the set of alleles to generate a respective normalized number of WBC-derived sequence reads corresponding to the allele;
wherein, for each allele in the set of alleles, the absolute difference is based on a difference between the normalized number of cfDNA sequence reads for the allele and the normalized number of WBC-derived sequence reads for the allele.

13. The system of claim 11, wherein the one or more processors are configured to:
generate a machine-learning or statistical classifier that generates a decision boundary on a coordinate space that separates a first set of data points that represent presence of microsatellite instability in sequence reads and a second set of data points that represent no presence of microsatellite instability in sequence reads; and
process the first distribution using the classifier to determine whether the first distribution belongs to the first set of data points or to the second set of data points; and
determine microsatellite instability responsive to the classifier classifying the first distribution as belonging to the first set of data points that represent presence of microsatellite instability.

## Patentansprüche

1. Computerimplementiertes Verfahren, umfassend:
das Empfangen, durch einen oder mehrere Prozessoren, einer Sequenzierungsvorrichtung der nächsten Generation von:
(i) einer Vielzahl von zellfreien DNA-Sequenz-Read-Paaren (cfDNA), die von einem Probanden stammen, wobei jeder cfDNA-Sequenz-Read aus der Vielzahl von cfDNA-Sequenz-Reads entweder einen vorwärtsgerichteten eindeutigen molekularen Identifikator (Unique Molecular Identifier, UMI) oder einen rückwärtsgerichteten UMI einschließt, und
(ii) einer Vielzahl von aus weißen Blutkörperchen (WBK) stammenden Sequenz-Read-Paaren, die von dem Probanden stammen, wobei jeder aus WBK stammende Sequenz-Read der Vielzahl von aus WBK stammenden Sequenz-Reads optional den vorwärtsgerichteten UMI oder den rückwärtsgerichteten UMI einschließt,
für jeden Mikrosatellitenlocus einer Vielzahl von Mikrosatellitenloci,
das Identifizieren eines ersten Teilsatzes der Vielzahl von cfDNA-Sequenz-Reads und eines zweiten Teilsatzes der Vielzahl der aus WBK stammenden Sequenz-Reads durch den einen oder die mehreren Prozessoren, wobei jeder Read in dem ersten Teilsatz und dem zweiten Teilsatz dem Mikrosatellitenlocus entspricht,
das Identifizieren eines Satzes von Allelen aus dem ersten Teilsatz und dem zweiten Teilsatz durch den einen oder die mehreren Prozessoren, wobei jedes Allel des Satzes von Allelen eine unterschiedliche Sequenz aufweist,
das Bestimmen einer Anzahl von cfDNA-Sequenzlesungen, die das Allel einschließen, für jedes Allel des Satzes von Allelen, durch den einen oder die mehreren Prozessoren, das Bestimmen einer Anzahl von aus WBK stammenden Sequenz-Reads, die das Allel einschließen, für jedes Allel des Satzes von Allelen, durch den einen oder mehrere Prozessoren,
das Bestimmen einer absoluten Differenz, basierend auf einer Differenz zwischen der Anzahl von cfDNA-Sequenz-Reads für das Allel und der Anzahl aus WBK stammenden Sequenz-Reads für das Allel, für jedes Allel in dem Satz von Allelen, durch den einen oder die mehreren Prozessoren,
das Bestimmen eines Abstandes, der auf einer Summe von absoluten Differenzen basiert, die mit allen Allelen in dem Satz von Allelen verbunden sind, für jeden Mikrosatellitenlocus aus der Vielzahl von Mikrosatellitenloci, durch den einen oder die mehreren Prozessoren,
das Erzeugen einer ersten Verteilung durch den einen oder die mehreren Prozessoren, die eine Anzahl von Mikrosatellitenloci angibt, deren Abstände innerhalb einer Gruppe von unterschiedlichen Abstandsintervallen liegen,
das Erzeugen, einer zweiten Verteilung durch den einen oder die mehreren Prozessoren, die eine Anzahl von Mikrosatellitenloci angibt, deren Abstände innerhalb der Gruppe von unterschiedlichen Abstandsintervallen liegen, wobei die zweite Verteilung von Abständen abgeleitet ist, die mit jedem Mikrosatellitenlocus der Vielzahl von Mikrosatellitenloci verbunden sind, die in einer Referenzprobe beobachtet wurden,
das Bestimmen, dass eine Anzahl von Mikrosatellitenloci in der ersten Verteilung oberhalb eines Schwellenwerts der Abstandsmetrik größer ist als eine Anzahl von Mikrosatellitenloci in der zweiten Verteilung oberhalb des Schwellenwerts der Abstandsmetrik durch den einen oder die mehreren Prozessoren, um ein Vorhandensein von Mikrosatelliteninstabilität in dem Probanden zu erkennen, und
das Speichern einer Assoziation zwischen dem Probanden und dem Vorhandensein einer Mikrosatelliten-Instabilität durch den einen oder die mehreren Prozessoren als Reaktion auf die Bestimmung in einer oder mehreren Datenstrukturen.

2. Computerimplementiertes Verfahren nach Anspruch 1, ferner umfassend:
das Normalisieren der Anzahl der cfDNA-Sequenz-Reads, die das Allel einschließen, für jedes Allel des Satzes von Allelen, durch den einen oder die mehreren Prozessoren, und zwar basierend auf einer Summe der Anzahl der cfDNA-Sequenz-Reads, die allen Allelen in dem Satz von Allelen entsprechen, um eine jeweilige normalisierte Anzahl von cfDNA-Sequenz-Reads zu erzeugen, die dem Allel entsprechen,
das Normalisieren der Anzahl der aus WBK stammenden Sequenzen, die das Allel einschließen, für jedes Allel des Satzes von Allelen, durch den einen oder die mehreren Prozessoren, und zwar basierend auf einer Summe der Anzahl der aus WBK stammenden Sequenz-Reads, die allen Allelen in dem Satz von Allelen entsprechen, um eine jeweilige normalisierte Anzahl von aus WBK stammenden Sequenz-Reads zu erzeugen, die dem Allel entsprechen,
wobei für jedes Allel in dem Satz von Allelen die absolute Differenz auf einer Differenz zwischen der normalisierten Anzahl von cfDNA-Sequenz-Reads für das Allel und der normalisierten Anzahl von aus WBK stammenden Sequenzlesungen für das Allel basiert, wobei optional die Summe der absoluten Differenzen, die mit allen Allelen in dem Satz von Allelen verbunden sind, auf einer Summe einer absoluten Differenz zwischen der normalisierten Anzahl von cfDNA-Sequenz-Reads und der normalisierten Anzahl von aus WBK stammenden Sequenz-Reads für jedes Allel in dem Satz von Allelen basiert.

3. Computerimplementiertes Verfahren nach einem der Ansprüche 1 bis 2, wobei die cfDNA-Probe Serum, Plasma, Schweiß, Tränen, Urin, Speichel, Synovialflüssigkeit, Lymphflüssigkeit, Aszitesflüssigkeit, Amnionflüssigkeit oder interstitielle Flüssigkeit ist oder wobei der Proband
unter dem Lynch-Syndrom leidet oder der Verdacht besteht, dass der Proband daran leidet;
mindestens eine Mutation in einem oder mehreren Mismatch-Reparaturgenen aufweist, die aus der Gruppe bestehend aus MSH2, MSH6, MLH1 und PMS2 ausgewählt sind; und/oder
an Eierstockkrebs, Brustkrebs, Darmkrebs, Lungenkrebs, Prostatakrebs, Magenkrebs, Bauchspeicheldrüsenkrebs, Gebärmutterhalskrebs, Leberkrebs, Blasenkrebs, Krebs der Harnwege, Schilddrüsenkrebs, Nierenkrebs, Karzinom, Melanom, Kopf- und Halskrebs oder einem Hirntumor leidet oder ein entsprechendes Risiko dafür aufweist.

4. Computerimplementiertes Verfahren nach einem der Ansprüche 1 bis 2 oder 4, ferner umfassend das Bestimmen des Vorhandenseins mindestens einer Mutation in einem Exon eines mit Krebs in Zusammenhang stehenden Gens, ausgewählt aus der Gruppe bestehend aus: AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3, GNA11, GNAQ, GNAS, H3F3A, HIST1H3B, HRAS, IDH1, IDH2, IKZF1, INPPL1, JAK1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL und XPO1, wobei die mindestens eine Mutation gegebenenfalls eine Deletion, eine Insertion, eine Translokation, eine Inversion, eine Kopienzahlvariante oder eine Punktmutation ist; und/oder
das Bestimmen des Vorhandenseins von mindestens einer genomischen Veränderung in einem Intron eines mit Krebs in Zusammenhang stehenden Gens, ausgewählt aus der Gruppe bestehend aus: ALK, BRAF, EGFR, ETV6, FGFR2, FGFR3, MET, NTRK1, RET und ROS1 oder eine Promoterregion von TERT.

5. Computerimplementiertes Verfahren nach Anspruch 1, ferner umfassend:
das Erzeugen eines maschinell lernenden oder statistischen Klassifizierers durch den einen oder die mehreren Prozessoren, der eine Entscheidungsgrenze in einem Koordinatenraum erzeugt, die einen ersten Satz von Datenpunkten, die das Vorhandensein von Mikrosatelliteninstabilität in Sequenz-Reads darstellen, und einen zweiten Satz von Datenpunkten, die kein Vorhandensein von Mikrosatelliteninstabilität in Sequenz-Reads darstellen, trennt;
das Verarbeiten der ersten Verteilung durch den einen oder die mehreren Prozessoren unter Verwendung des Klassifizierers, um zu bestimmen, ob die erste Verteilung zu dem ersten Satz von Datenpunkten oder zu dem zweiten Satz von Datenpunkten gehört; und
das Bestimmen der Mikrosatelliten-Instabilität durch den einen oder die mehreren Prozessoren in Reaktion darauf, dass der Klassifizierer die erste Verteilung als zu dem ersten Satz von Datenpunkten gehörend klassifiziert, die das Vorhandensein einer Mikrosatelliten-Instabilität darstellen, wobei der Klassifizierer optional eine Support-Vektor-Maschine (SVM) einschließt.

6. Verfahren zum Überwachen des Fortschreitens von Krebs bei einem Probanden, umfassend:
das Nachweisen des Vorhandenseins von Mikrosatelliteninstabilität in einer zellfreien DNA-Probe (cfDNA), die von dem Probanden erhalten wurde, unter Verwendung des computerimplementierten Verfahrens nach einem der vorangehenden Ansprüche, wobei gegebenenfalls
das Fortschreiten des Krebses Metastasen in sekundären Organen, eine Zunahme des Tumorvolumens oder der Tumorlast oder eine erhöhte Tumorproliferation einschließt, oder
die cfDNA-Probe keine Mutation in einem mit Krebs in Zusammenhang stehenden Gen enthält, das aus der Gruppe ausgewählt ist, die aus AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3, GNA11, GNAQ, GNAS, H3F3A, HIST1H3B, HRAS, IDH1, IDH2, IKZF1, INPPL1, JAK1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL, und XPO1 besteht, oder
die cfDNA-Probe keine genomische Veränderung in einem mit Krebs in Zusammenhang stehenden Gen aufweist, das aus der Gruppe ausgewählt ist, die aus ALK, BRAF, EGFR, ETV6, FGFR2, FGFR3, MET, NTRK1, RET und ROS1 oder einer Promotorregion von TERT besteht, oder
der Proband keine nachweisbaren Tumore hat.

7. Verfahren zum Bestimmen der Wirksamkeit einer Therapie bei einem Probanden mit einem MSI-High-Tumor, umfassend:
das Bereitstellen einer ersten zellfreien DNA-Probe (cfDNA), die von dem Probanden nach der Verabreichung der Therapie erhalten wurde,
das Nachweisen des Vorhandenseins einer Mikrosatelliten-Instabilität in der ersten cfDNA-Probe unter Verwendung des computerimplementierten Verfahrens nach einem der Ansprüche 1 bis 5, und
das Bestimmen, dass die Therapie wirksam ist, wenn die erste cfDNA-Probe im Vergleich zu derjenigen, die in einer Kontrollprobe beobachtet wurde, die von dem Probanden vor der Verabreichung der Therapie erhalten wurde, eine Verschiebung in Richtung einer Abstandsmetrik zeigt, die mit Mikrosatelliten-Stabilität (MSS) verbunden ist.

8. Verfahren nach Anspruch 7, wobei es sich bei der Therapie um eine oder mehrere der folgenden Therapien handelt: Strahlentherapie, Chemotherapie, Chirurgie, Immuntherapie oder Operation.

9. Verfahren nach Anspruch 8, wobei die Chemotherapie die Verabreichung eines oder mehrerer chemotherapeutischer Mittel einschließt, die aus der Gruppe ausgewählt sind, die aus Abraxane, Capecitabin, Erlotinib, Fluorouracil (5-FU), Gemcitabin, Irinotecan, Leucovorin, Nab-Paclitaxel, Cisplatin, Irinotecan, Docetaxel, Oxaliplatin, Tipifarnib, Everolimus, Sunitinib, Dovitinib, Ruxolitinib, pegylierte Hyaluronidase, Pemetrexed, Folinsäure, Paclitaxel, MK2206, GDC-0449, IPI-926, Gamma-Sekretase/RO4929097, M402, und LY293111 besteht.

10. Verfahren nach Anspruch 8, wobei die Immuntherapie die Verabreichung eines oder mehrerer Wirkstoffe einschließt, die aus der Gruppe ausgewählt sind, die aus Immun-Checkpoint-Inhibitoren (z. B. Antikörpern gegen CTLA-4, PD-1, PD-L1), Ipilimumab, 90Y-Clivatuzumab Tetraxetan, Pembrolizumab, Nivolumab, Trastuzumab, Cixutumumab, Ganitumab, Demcizumab, Cetuximab, Nimotuzumab, Dalotuzumab, Sipuleucel-T, CRS-207 und GVAX besteht.

11. System, umfassend:
einen oder mehrere Prozessoren, die konfiguriert sind, zum:
Empfangen von einer Sequenzierungsvorrichtung der nächsten Generation von:
(i) einer Vielzahl von zellfreien DNA-Sequenz-Read-Paaren (cfDNA), die von einem Probanden stammen, wobei jeder cfDNA-Sequenz-Read aus der Vielzahl von cfDNA-Sequenz-Reads entweder einen vorwärtsgerichteten eindeutigen molekularen Identifikator (Unique Molecular Identifier, UMI) oder einen rückwärtsgerichteten UMI einschließt, und
(ii) einer Vielzahl von aus weißen Blutkörperchen (WBK) stammenden Sequenz-Reads, die von dem Probanden stammen, wobei jeder aus WBK stammende Sequenz-Read der Vielzahl von aus WBK stammenden Sequenz-Reads optional den vorwärtsgerichteten UMI oder den rückwärtsgerichteten UMI einschließt,
für jeden Mikrosatellitenlocus einer Vielzahl von Mikrosatellitenloci,
Identifizieren eines ersten Teilsatzes der Vielzahl von cfDNA-Sequenz-Reads und eines zweiten Teilsatzes der Vielzahl der aus WBK stammenden Sequenz-Reads durch den einen oder die mehreren Prozessoren, wobei jeder Read in dem ersten Teilsatz und dem zweiten Teilsatz dem Mikrosatellitenlocus entspricht,
Identifizieren eines Satzes von Allelen aus dem ersten Teilsatz und dem zweiten Teilsatz, wobei jedes Allel des Satzes von Allelen eine unterschiedliche Sequenz aufweist,
Bestimmen einer Anzahl von cfDNA-Sequenzlesungen, die das Allel einschließen, für jedes Allel des Satzes von Allelen,
Bestimmen einer Anzahl von aus WBK stammenden Sequenz-Reads, die das Allel einschließen, für jedes Allel des Satzes von Allelen,
Bestimmen einer absoluten Differenz, basierend auf einer Differenz zwischen der Anzahl von cfDNA-Sequenz-Reads für das Allel und der Anzahl aus WBK stammenden Sequenz-Reads für das Allel, für jedes Allel in dem Satz von Allelen, Bestimmen eines Abstandes, der auf einer Summe von absoluten Differenzen basiert, die mit allen Allelen in dem Satz von Allelen verbunden sind, für jeden Mikrosatellitenlocus aus der Vielzahl von Mikrosatellitenloci,
Erzeugen einer ersten Verteilung, die eine Anzahl von Mikrosatellitenloci angibt, deren Abstände innerhalb einer Gruppe von unterschiedlichen Abstandsintervallen liegen,
Erzeugen, einer zweiten Verteilung, die eine Anzahl von Mikrosatellitenloci angibt, deren Abstände innerhalb der Gruppe von unterschiedlichen Abstandsintervallen liegen, wobei die zweite Verteilung von Abständen abgeleitet ist, die mit jedem Mikrosatellitenlocus der Vielzahl von Mikrosatellitenloci verbunden sind, die in einer Referenzprobe beobachtet wurden,
Bestimmen, dass eine Anzahl von Mikrosatellitenloci in der ersten Verteilung oberhalb eines Schwellenwertes der Abstandsmetrik größer ist als eine Anzahl von Mikrosatellitenloci in der zweiten Verteilung oberhalb des Schwellenwertes der Abstandsmetrik, um ein Vorhandensein von Mikrosatelliten-Instabilität in dem Probanden zu erkennen, und
Speichern einer Assoziation zwischen dem Probanden und dem Vorhandensein einer Mikrosatelliten-Instabilität als Reaktion auf die Bestimmung in einer oder mehreren Datenstrukturen.

12. System nach Anspruch 11, wobei der eine oder die mehreren Prozessoren konfiguriert sind zum:
Normalisieren der Anzahl der cfDNA-Sequenz-Reads, die das Allel einschließen, für jedes Allel des Satzes von Allelen, und zwar basierend auf einer Summe der Anzahl der cfDNA-Sequenz-Reads, die allen Allelen in dem Satz von Allelen entsprechen, um eine jeweilige normalisierte Anzahl von cfDNA-Sequenz-Reads zu erzeugen, die dem Allel entsprechen,
Normalisieren der Anzahl der aus WBK stammenden Sequenzen, die das Allel einschließen, für jedes Allel des Satzes von Allelen, und zwar basierend auf einer Summe der Anzahl der aus WBK stammenden Sequenz-Reads, die allen Allelen in dem Satz von Allelen entsprechen, um eine jeweilige normalisierte Anzahl von aus WBK stammenden Sequenz-Reads zu erzeugen, die dem Allel entsprechen,
wobei für jedes Allel in dem Satz von Allelen die absolute Differenz auf einer Differenz zwischen der normalisierten Anzahl von cfDNA-Sequenz-Reads für das Allel und der normalisierten Anzahl von aus WBK stammenden Sequenz-Reads für das Allel basiert.

13. System nach Anspruch 11, wobei der eine oder die mehreren Prozessoren konfiguriert sind zum:
Erzeugen eines maschinell lernenden oder statistischen Klassifizierers, der eine Entscheidungsgrenze in einem Koordinatenraum erzeugt, die einen ersten Satz von Datenpunkten, die das Vorhandensein von Mikrosatelliten-Instabilität in Sequenz-Reads darstellen, und einen zweiten Satz von Datenpunkten, die kein Vorhandensein von Mikrosatelliten-Instabilität in Sequenz-Reads darstellen, trennt, und
Verarbeiten der ersten Verteilung unter Verwendung des Klassifizierers, um zu bestimmen, ob die erste Verteilung zu dem ersten Satz von Datenpunkten oder zu dem zweiten Satz von Datenpunkten gehört, und
Bestimmen der Mikrosatelliten-Instabilität in Reaktion darauf, dass der Klassifizierer die erste Verteilung als zu dem ersten Satz von Datenpunkten gehörend klassifiziert, die das Vorhandensein von Mikrosatelliten-Instabilität darstellen.

## Revendications

1. Procédé implémenté par ordinateur, comprenant :
la réception, par un ou plusieurs processeurs, d'un dispositif de séquençage de nouvelle génération :
(i) une pluralité de paires de lectures de séquences d'ADN libre circulant (ADNcf) provenant d'un sujet, chaque lecture de séquence d'ADNcf parmi la pluralité de lectures de séquences d'ADNcf comportant soit un identifiant moléculaire unique (UMI) direct, soit un UMI inverse, et
(ii) une pluralité de paires de lectures de séquences dérivées de leucocytes provenant du sujet, chaque lecture de séquence dérivée de leucocytes parmi la pluralité de lectures de séquences dérivées de leucocytes comportant éventuellement l'UMI direct ou l'UMI inverse ;
pour chaque locus microsatellite parmi une pluralité de loci microsatellites,
l'identification, par l'un ou plusieurs processeurs, d'un premier sous-ensemble parmi la pluralité de lectures de séquences d'ADNcf et d'un second sous-ensemble parmi la pluralité de lectures de séquences dérivées de leucocytes, chaque lecture dans le premier sous-ensemble et le second sous-ensemble correspond au locus microsatellite ;
l'identification, par l'un ou plusieurs processeurs, à partir du premier sous-ensemble et du second sous-ensemble, d'un ensemble d'allèles, chaque allèle parmi l'ensemble d'allèles ayant une séquence distincte ;
la détermination, par l'un ou plusieurs processeurs, pour chaque allèle parmi l'ensemble d'allèles, d'un nombre de lectures de séquences d'ADNcf qui comportent l'allèle ;
la détermination, par l'un ou plusieurs processeurs, pour chaque allèle parmi l'ensemble d'allèles, d'un nombre de lectures de séquences dérivées de leucocytes qui comportent l'allèle ;
la détermination, par l'un ou plusieurs processeurs, pour chaque allèle dans l'ensemble d'allèles, d'une différence absolue sur la base d'une différence entre le nombre de lectures de séquences d'ADNcf pour l'allèle et le nombre de lectures de séquences dérivées de leucocytes pour l'allèle,
la détermination, par l'un ou plusieurs processeurs, pour chaque locus microsatellite parmi la pluralité de loci microsatellites, d'une distance sur la base d'une somme de différences absolues associées à tous les allèles dans l'ensemble d'allèles ;
la génération, par l'un ou plusieurs processeurs, d'une première distribution indiquant un nombre de loci microsatellites ayant des distances à l'intérieur d'un groupe d'intervalles de distance distincts ;
la génération, par l'un ou plusieurs processeurs, d'une seconde distribution indiquant un nombre de loci microsatellites ayant des distances à l'intérieur du groupe d'intervalles de distance distincts, la seconde distribution étant dérivée des distances associées à chaque locus microsatellite parmi la pluralité de loci microsatellites observés dans un échantillon de référence ;
la détermination, par l'un ou plusieurs processeurs, qu'un nombre de loci microsatellites dans la première distribution au-dessus d'une métrique de distance seuil est supérieur à un nombre de loci microsatellites dans la seconde distribution au-dessus de la métrique de distance seuil pour détecter une présence d'instabilité des microsatellites chez le sujet ; et
le stockage, par l'un ou plusieurs processeurs, en réponse à la détermination, dans une ou plusieurs structures de données, d'une association entre le sujet et la présence d'une instabilité des microsatellites.

2. Procédé implémenté par ordinateur selon la revendication 1, comprenant en outre :
la normalisation, par l'un ou plusieurs processeurs, pour chaque allèle parmi l'ensemble d'allèles, du nombre de lectures de séquences d'ADNcf qui comportent l'allèle sur la base d'une somme du nombre de lectures de séquences d'ADNcf correspondant à tous les allèles dans l'ensemble d'allèles pour générer un nombre normalisé respectif de lectures de séquences d'ADNcf correspondant à l'allèle ;
la normalisation, par l'un ou plusieurs processeurs, pour chaque allèle parmi l'ensemble d'allèles, du nombre de séquences dérivées de leucocytes qui comportent l'allèle sur la base d'une somme du nombre de lectures de séquences dérivées de leucocytes correspondant à tous les allèles dans l'ensemble d'allèles pour générer un nombre normalisé respectif de lectures de séquences dérivées de leucocytes correspondant à l'allèle ;
dans lequel, pour chaque allèle dans l'ensemble d'allèles, la différence absolue repose sur une différence entre le nombre normalisé de lectures de séquences d'ADNcf pour l'allèle et le nombre normalisé de lectures de séquences dérivées de leucocytes pour l'allèle, éventuellement dans lequel la somme des différences absolues associées à tous les allèles dans l'ensemble d'allèles repose sur une somme d'une différence absolue entre le nombre normalisé de lectures de séquences d'ADNcf et le nombre normalisé de lectures de séquences dérivées de leucocytes pour chaque allèle dans l'ensemble d'allèles.

3. Procédé implémenté par ordinateur selon l'une quelconque des revendications 1 à 2, dans lequel l'échantillon d'ADNcf est du sérum, du plasma, de la sueur, des larmes, de l'urine, de la salive, du liquide synovial, du liquide lymphatique, du liquide d'ascite, du liquide amniotique ou du liquide interstitiel ou dans lequel le sujet
souffre ou est suspecté d'être atteint du syndrome de Lynch ;
présente au moins une mutation dans un ou plusieurs gènes de réparation des mésappariements sélectionnés parmi le groupe constitué de MSH2, MSH6, MLH1 et PMS2 ; et/ou souffre ou présente un risque de cancer de l'ovaire, de cancer du sein, de cancer colorectal, de cancer du poumon, de cancer de la prostate, de cancer gastrique, de cancer du pancréas, de cancer du col de l'utérus, de cancer du foie, de cancer de la vessie, de cancer des voies urinaires, de cancer de la thyroïde, de cancer du rein, de carcinome, de mélanome, de cancer de la tête et du cou ou de cancer du cerveau.

4. Procédé implémenté par ordinateur selon l'une quelconque des revendications 1 à 2 ou 4, comprenant en outre la détermination de la présence d'au moins une mutation dans un exon d'un gène lié au cancer sélectionné dans le groupe constitué de : AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3,
GNA11, GNAQ, GNAS, H3F3A, HIST1H3B, HRAS, IDH1, IDH2, IKZF1, INPPL1, JAK1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL, et XPO1, éventuellement dans lequel l'au moins une mutation est une délétion, une insertion, une translocation, une inversion, une variante du nombre de copies ou une mutation ponctuelle ; et/ou
la détermination de la présence d'au moins une altération génomique dans un intron d'un gène lié au cancer sélectionné parmi le groupe constitué de : ALK, BRAF, EGFR. ETV6, FGFR2, FGFR3, MET, NTRK1, RET et ROS1 ou une région promotrice de TERT.

5. Procédé implémenté par ordinateur selon la revendication 1, comprenant en outre :
la génération, par l'un ou plusieurs processeurs, d'un classificateur d'apprentissage automatique ou statistique qui génère une limite de décision sur un espace de coordonnées qui sépare un premier ensemble de points de données qui représentent la présence d'une instabilité des microsatellites dans les lectures de séquences et un second ensemble de points de données qui représentent l'absence d'instabilité des microsatellites dans les lectures de séquences ;
le traitement, par l'un ou plusieurs processeurs, de la première distribution à l'aide du classificateur pour déterminer si la première distribution appartient au premier ensemble de points de données ou au second ensemble de points de données ; et
la détermination, par l'un ou plusieurs processeurs, de l'instabilité des microsatellites en réponse au classificateur classant la première distribution comme appartenant au premier ensemble de points de données qui représentent la présence d'une instabilité des microsatellites, éventuellement dans lequel le classificateur comporte une machine à vecteurs de support (SVM).

6. Procédé permettant de surveiller la progression du cancer chez un sujet, comprenant :
la détection de la présence d'une instabilité des microsatellites dans un échantillon d'ADN libre circulant (ADNcf) obtenu auprès du sujet à l'aide du procédé implémenté par ordinateur selon l'une quelconque des revendications précédentes, éventuellement dans lequel
la progression du cancer comporte des métastases aux organes secondaires, des augmentations du volume tumoral ou de la charge tumorale, ou une prolifération tumorale accrue, ou
l'échantillon d'ADNcf ne comprend pas de mutation dans un gène lié au cancer sélectionné parmi le groupe constitué de : AKT1, ALK, APC, AR, ARAF, ARID1A, ARID2, ATM, B2M, BCL2, BCOR, BRAF, BRCA1, BRCA2, CARD11, CBFB, CCND1, CDH1, CDK4, CDKN2A, CIC, CREBBP, CTCF, CTNNB1, DICER1, DIS3, DNMT3A, EGFR, EIF1AX, EP300, ERBB2, ERBB3, ERCC2, ESR1, EZH2, FBXW7, FGFR1, FGFR2, FGFR3, FGFR4, FLT3, FOXA1, FOXL2, FOXO1, FUBP1, GATA3, GNA11, GNAQ, GNAS, H3F3A, HIST1H3B, HRAS, IDH1, IDH2, IKZF1, INPPL1, JAK1, KDM6A, KEAP1, KIT, KNSTRN, KRAS, MAP2K1, MAPK1, MAX, MED12, MET, MLH1, MSH2, MSH3, MSH6, MTOR, MYC, MYCN, MYD88, MYOD1, NF1, NFE2L2, NOTCH1, NRAS, NTRK1, NTRK2, NTRK3, NUP93, PAK7, PDGFRA, PIK3CA, PIK3CB, PIK3R1, PIK3R2, PMS2, POLE, PPP2R1A, PPP6C, PRKCI, PTCH1, PTEN, PTPN11, RAC1, RAF1, RB1, RET, RHOA, RIT1, ROS1, RRAS2, RXRA, SETD2, SF3B1, SMAD3, SMAD4, SMARCA4, SMARCB1, SOS1, SPOP, STAT3, STK11, STK19, TCF7L2, TERT, TGFBR1, TGFBR2, TP53, TP63, TSC1, TSC2, U2AF1, VHL, et XPO1 ou
l'échantillon d'ADNcf ne comprend pas d'altération génomique dans un gène lié au cancer sélectionné dans le groupe constitué de : ALK, BRAF, EGFR. ETV6, FGFR2, FGFR3, MET, NTRK1, RET et ROS1 ou une région promotrice de TERT, ou
le sujet ne présente aucune tumeur détectable.

7. Procédé permettant de déterminer l'efficacité d'une thérapie chez un sujet atteint d'une tumeur à haute instabilité des microsatellites, comprenant :
la fourniture d'un premier échantillon d'ADN libre circulant (ADNcf) obtenu auprès du sujet après que la thérapie a été administrée ;
la détection de la présence d'une instabilité des microsatellites dans le premier échantillon d'ADNcf à l'aide du procédé implémenté par ordinateur selon l'une quelconque des revendications 1 à 5 ; et
la détermination que la thérapie est efficace lorsque le premier échantillon d'ADNcf montre une évolution vers une métrique de distance qui est associée à la stabilité des microsatellites (MSS) par rapport à celle observée dans un échantillon témoin obtenu auprès du sujet avant l'administration de la thérapie.

8. Procédé selon la revendication 7, dans lequel la thérapie est une ou plusieurs parmi la radiothérapie, la chimiothérapie, la chirurgie, l'immunothérapie ou la chirurgie.

9. Procédé selon la revendication 8, dans lequel la chimiothérapie comporte l'administration d'un ou plusieurs agents chimiothérapeutiques sélectionnés parmi le groupe constitué d'abraxane, de capécitabine, d'erlotinib, de fluorouracile (5-FU), de gemcitabine, d'irinotécan, de leucovorine, de nab-paclitaxel, de cisplatine, d'irinotécan, de docétaxel, d'oxaliplatine, de tipifarnib, d'évérolimus, de sunitinib, de dovitinib, de ruxolitinib, d'hyaluronidase pégylée, de pémétrexed, d'acide folinique, de paclitaxel, de MK2206, de GDC-0449, de IPI-926, de gamma-sécrétase/RO4929097, de M402 et de LY293111.

10. Procédé selon la revendication 8, dans lequel l'immunothérapie comporte l'administration d'un ou plusieurs agents sélectionnés dans le groupe constitué des inhibiteurs de points de contrôle immunitaires (par exemple, des anticorps ciblant CTLA-4, PD-1, PD-L1), d'ipilimumab, de 90Y-Clivatuzumab tétraxétan, de pembrolizumab, de nivolumab, de trastuzumab, de cixutumumab, de ganitumab, de demcizumab, de cetuximab, de nimotuzumab, de dalotuzumab, de sipuleucel-T, de CRS-207 et de GVAX.

11. Système, comprenant :
un ou plusieurs processeurs, configurés pour :
recevoir d'un dispositif de séquençage de nouvelle génération :
(i) une pluralité de paires de lectures de séquences d'ADN libre circulant (ADNcf) provenant d'un sujet, chaque lecture de séquence d'ADNcf parmi la pluralité de lectures de séquences d'ADNcf comportant soit un identifiant moléculaire unique (UMI) direct, soit un UMI inverse, et
(ii) une pluralité de lectures de séquences dérivées de leucocytes provenant du sujet, chaque lecture de séquence dérivée de leucocytes parmi la pluralité de lectures de séquences dérivées de leucocytes comportant éventuellement l'UMI direct ou l'UMI inverse ;
pour chaque locus microsatellite parmi une pluralité de loci microsatellites,
identifier un premier sous-ensemble parmi la pluralité de lectures de séquences d'ADNcf et un second sous-ensemble parmi la pluralité de lectures de séquences dérivées de leucocytes, chaque lecture dans le premier sous-ensemble et le second sous-ensemble correspond au locus microsatellite ;
identifier à partir du premier sous-ensemble et du second sous-ensemble, un ensemble d'allèles, chaque allèle parmi l'ensemble d'allèles ayant une séquence distincte ;
déterminer, pour chaque allèle parmi l'ensemble d'allèles, un nombre de lectures de séquences d'ADNcf qui comportent l'allèle ;
déterminer, pour chaque allèle parmi l'ensemble d'allèles, un nombre de lectures de séquences dérivées de leucocytes qui comportent l'allèle ;
déterminer, pour chaque allèle dans l'ensemble d'allèles, une différence absolue sur la base d'une différence entre le nombre de lectures de séquences d'ADNcf pour l'allèle et le nombre de lectures de séquences dérivées de leucocytes pour l'allèle,
déterminer, pour chaque locus microsatellite parmi la pluralité de loci microsatellites, une distance sur la base d'une somme de différences absolues associées à tous les allèles dans l'ensemble d'allèles ;
générer une première distribution indiquant un nombre de loci microsatellites ayant des distances à l'intérieur d'un groupe d'intervalles de distance distincts ;
générer une seconde distribution indiquant un nombre de loci microsatellites ayant des distances à l'intérieur du groupe d'intervalles de distance distincts, la seconde distribution étant dérivée des distances associées à chaque locus microsatellite parmi la pluralité de loci microsatellites observés dans un échantillon de référence ;
déterminer qu'un nombre de loci microsatellites dans la première distribution au-dessus d'une métrique de distance seuil est supérieur à un nombre de loci microsatellites dans la seconde distribution au-dessus de la métrique de distance seuil pour détecter une présence d'instabilité des microsatellites chez le sujet ; et
stocker, en réponse à la détermination, dans une ou plusieurs structures de données, une association entre le sujet et la présence d'une instabilité des microsatellites.

12. Système selon la revendication 11, dans lequel l'un ou plusieurs processeurs sont configurés pour :
normaliser, pour chaque allèle parmi l'ensemble d'allèles, le nombre de lectures de séquences d'ADNcf qui comportent l'allèle sur la base d'une somme du nombre de lectures de séquences d'ADNcf correspondant à tous les allèles dans l'ensemble d'allèles pour générer un nombre normalisé respectif de lectures de séquences d'ADNcf correspondant à l'allèle ;
normaliser, pour chaque allèle parmi l'ensemble d'allèles, le nombre de séquences dérivées de leucocytes qui comportent l'allèle sur la base d'une somme du nombre de lectures de séquences dérivées de leucocytes correspondant à tous les allèles dans l'ensemble d'allèles pour générer un nombre normalisé respectif de lectures de séquences dérivées de leucocytes correspondant à l'allèle ;
dans lequel, pour chaque allèle dans l'ensemble d'allèles, la différence absolue repose sur une différence entre le nombre normalisé de lectures de séquences d'ADNcf pour l'allèle et le nombre normalisé de lectures de séquences dérivées de leucocytes pour l'allèle.

13. Système selon la revendication 11, dans lequel l'un ou plusieurs processeurs sont configurés pour :
générer un classificateur d'apprentissage automatique ou statistique qui génère une limite de décision sur un espace de coordonnées qui sépare un premier ensemble de points de données qui représentent la présence d'une instabilité des microsatellites dans les lectures de séquences et un second ensemble de points de données qui représentent l'absence d'instabilité des microsatellites dans les lectures de séquences ; et
traiter la première distribution à l'aide du classificateur pour déterminer si la première distribution appartient au premier ensemble de points de données ou au second ensemble de points de données ; et
déterminer l'instabilité des microsatellites en réponse au classificateur classant la première distribution comme appartenant au premier ensemble de points de données qui représentent la présence d'une instabilité des microsatellites.
